# EUROPÄISCHE PATENTSCHRIFT

(11) **EP 0 793 645 B1**
(45) Veröffentlichungstag und Bekanntmachung des Hinweises auf die Patenterteilung: **28.04.1999**
(21) Anmeldenummer: 95938441.3
(22) Anmeldetag: 10.11.1995
(51) Int. Cl.: C07C 271/28, C07C 275/64, A01N 47/20, A01N 47/40

(54) **IMINOOXYMETHYLENANILIDE, VERFAHREN UND ZWISCHENPRODUKTE ZU IHRER HERSTELLUNG UND SIE ENTHALTENDE SCHÄDLINGSBEKÄMFUNGSMITTEL**
IMINOOXYMETHYLENE ANILIDES, PROCESS AND INTERMEDIATE PRODUCTS FOR PREPARING THE SAME AND PESTICIDES CONTAINING THE SAME
ANILIDES D'IMINOOXYMETHYLENE, PROCEDE ET PRODUITS INTERMEDIAIRES DE PREPARATION DE CELLES-CI, ET PESTICIDES LES CONTENANT

(30) Priorität: 23.11.1994 DE 4441674
(43) Veröffentlichungstag der Anmeldung: 10.09.1997
(73) Patentinhaber: BASF AKTIENGESELLSCHAFT, 67056 Ludwigshafen (DE)
(72) Erfinder: MÜLLER, Bernd, D-67227 Frankenthal (DE); MÜLLER, Ruth, D-67159 Friedelsheim (DE); BAYER, Herbert, D-68159 Mannheim (US); SAUTER, Hubert, D-68167 Mannheim (DE); RÖHL, Franz, D-67105 Schifferstadt (DE); AMMERMANN, Eberhard, D-64646 Heppenheim (DE); LORENZ, Gisela, D-67434 Hambach (DE); GÖTZ, Norbert, D-67547 Worms (DE)
(86) Internationale Anmeldenummer: EP9504428
(87) Internationale Veröffentlichungsnummer: WO9616030

(56) Entgegenhaltungen:
- EP-A- 0 619 301
- WO-A-93/15046

## Beschreibung

Die vorliegende Erfindung betrifft Iminooximethylenanilide der Formel I 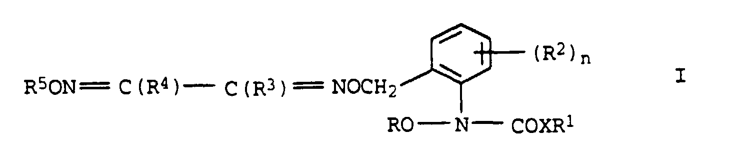 in der die Substituenten und der Index die folgende Bedeutung haben:
- R: Wasserstoff,
ggf. subst. Alkyl, Alkenyl, Alkinyl, Cycloalkyl, Cycloalkenyl, Cycloalkinyl, Alkylcarbonyl oder Alk-oxycarbonyl;
- R¹: Alkyl, Alkenyl, Alkinyl, Cycloalkyl oder Cycloalkenyl und für den Fall, daß X für NR^{a} steht, zusätzlich Wasserstoff;
- X: eine direkte Bindung, O oder NR^{a};
- R^{a}: Wasserstoff, Alkyl, Alkenyl, Alkinyl, Cycloalkyl oder Cycloalkenyl;
- R²: Cyano, Nitro, Trifluormethyl, Halogen, C₁-C₄-Alkyl oder C₁-C₄-Alkoxy;
- n: 0, 1 oder 2, wobei die Reste R² verschieden sein können, wenn n für 2 steht;
- R³: Wasserstoff, Hydroxy, Cyano, Cyclopropyl, Trifluormethyl, Halogen, C₁-C₆-Alkyl, C₁-C₆-Alkoxy oder C₁-C₆-Alkylthio;
- R⁴: Wasserstoff, Cyano, Nitro, Hydroxy, Amino, Halogen,
C₁-C₆-Alkyl, C₁-C₆-Alkoxy, C₁-C₆-Alkylthio, C₁-C₆-Alkylamino, Di-C₁-C₆-alkylamino, C₂-C₆-Alkenyl, C₂-C₆-Alkenyloxy, C₂-C₆-Alkenylthio, C₂-C₆-Alkenylamino, N-C₂-C₆-Alkenyl-N-C₁-C₆-alkylamino, C₂-C₆-Alkinyl, C₂-C₆-Alkinyloxy, C₂-C₆-Alkinylthio, C₂-C₆-Alkinylamino, N-C₂-C₆-Alkinyl-N-C₁-C₆-alkylamino, wobei die Kohlenwasserstoffreste dieser Gruppen partiell oder vollständig halogeniert sein können oder einen bis drei der folgenden Reste tragen können: Cyano, Nitro, Hydroxy, Merkapto,Amino, Carboxyl, Aminocarbonyl, Aminothiocarbonyl, Halogen, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₁-C₆-Alkylsulfonyl, C₁-C₆-Alkylsulfoxyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₂-C₆-Alkenyloxy, C₃-C₆-Cycloalkyl, C₃-C₆-Cycloalkyloxy, Heterocyclyl, Heterocyclyloxy, Aryl, Aryloxy, Aryl-C₁-C₄-alkoxy, Arylthio, Aryl-C₁-C₄-alkylthio, Hetaryl, Hetaryloxy, Hetaryl-C₁-C₄-alkoxy, Hetarylthio, Hetaryl-C₁-C₄-alkylthio, wobei die cyclischen Reste ihrerseits partiell oder vollständig halogeniert sein können und/oder ein bis drei der folgenden Gruppen tragen können: Cyano, Nitro, Hydroxy, Mercapto, Amino, Carboxyl, Aminocarbonyl, Aminothiocarbonyl, C₁-C₆-Alkyl, C₁-C₆-Halogenalkyl, C₁-C₆-Alkylsulfonyl, C₁-C₆-Alkylsulfoxyl, C₃C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyloxy, Aryl, Aryloxy, Arylthio, Hetaryl, Hetaryloxy, Hetarylthio und C (=NOR⁶) -Aₙ-R⁷;
C₃-C₆-Cycloalkyl, C₃-C₆-Cycloalkyloxy, C₃-C₆-Cycloalkylthio, C₃-C₆-Cycloalkylamino, N-C₃-C₆-Cycloalkyl-N-C₁-C₆-alkylamino, C₃-C₆-Cycloalkenyl, C₃-C₆-Cycloalkenyloxy, C₃-C₆-Cycloalkenylthio, C₃-C₆-Cycloalkenylamino, N-C₃-C₆-Cycloalkenyl-N-C₁-C₆-alkylamino, Heterocyclyl, Heterocyclyloxy, Heterocyclylthio, Heterocyclylamino, N-Heterocyclyl-N-C₁-C₆-alkylamino, Aryl, Aryloxy, Arylthio, Arylamino, N-Aryl-N-C₁-C₆-alkylamino, Hetaryl, Hetaryloxy, Hetarylthio, Hetarylamino, N-Hetaryl-N-C₁-C₆-alkylamino, wobei die cyclischen Reste partiell oder vollständig halogeniert sein können oder einen bis drei der folgenden Gruppen tragen können: Cyano, Nitro, Hydroxy, Mercapto, Amino, Carboxyl, Aminocarbonyl, Aminothiocarbonyl, Halogen, C₁-C₆-Alkyl, C₁-C₆-Halogenalkyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyloxy, Aryl, Aryloxy, Hetaryl und Hetaryloxy;
- R⁵: Wasserstoff,
C₁-C₁₀-Alkyl, C₃-C₆-Cycloalkyl, C₂-C₁₀-Alkenyl, C₂-C₁₀-Alkinyl, C₁-C₁₀-Alkylcarbonyl, C₂-C₁₀-Alkenylcarbonyl, C₃-C₁₀-Alkinylcarbonyl oder C₁-C₁₀-Alkylsulfonyl, wobei diese Reste partiell oder vollständig halogeniert sein können oder einen bis drei der folgenden Gruppen tragen können: Cyano, Nitro, Hydroxy, Mercapto, Amino, Carboxyl, Aminocarbonyl, Aminothiocarbonyl, Halogen, C₁-C₆-Alkyl, C₁-C₆-Halogenalkyl, C₁-C₆-Alkylsulfonyl, C₁-C₆-Alkylsulfoxyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, C₃-C₆-Cycloalkyl, C₃-C₆-Cycloalkyloxy, Heterocyclyl, Heterocyclyloxy, Benzyl, Benzyloxy, Aryl, Aryloxy, Arylthio, Hetaryl, Hetaryloxy und Hetarylthio, wobei die cyclischen Gruppen ihrerseits partiell oder vollständig halogeniert sein können oder einen bis drei der folgenden Gruppen tragen können: Cyano, Nitro, Hydroxy, Mercapto, Amino, Carboxyl, Aminocarbonyl, Aminothiocarbonyl, Halogen, C₁-C₆-Alkyl, C₁-C₆-Halogenalkyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkyloxycarbonyl, C₁-C₆-Alkylthio, C₁-C₆-Alkylamino, Di-C₁-C₆-Alkylamino, C₁-C₆-Alkylaminocarbonyl, Di-C₁-C₆-Alkylaminocarbonyl, C₁-C₆-Alkylaminothiocarbonyl, Di-C₁-C₆-Alkylaminothiocarbonyl, C₂-C₆-Alkenyl, C₂-C₆-Alkenyloxy, Benzyl, Benzyloxy, Aryl, Aryloxy, Arylthio, Hetaryl, Hetaryloxy, Hetarylthio oder C(=NOR⁶)-Aₙ-R⁷;
Aryl, Arylcarbonyl, Arylsulfonyl, Hetaryl, Hetarylcarbonyl oder Hetarylsulfonyl, wobei diese Reste partiell oder vollständig halogeniert sein können oder einen bis drei der folgenden Gruppen tragen können: Cyano, Nitro, Hydroxy, Mercapto, Amino, Carboxyl, Aminocarbonyl, Aminothiocarbonyl, Halogen, C₁-C₆-Alkyl, C₁-C₆-Halogenalkyl, C₁-C₆-Alkylcarbonyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkyloxycarbonyl, C₁-C₆-Alkylthio, C₁-C₆-Alkylamino, Di-C₁-C₆-Alkylamino, C₁-C₆-Alkylaminocarbonyl, Di-C₁-C₆-Alkylaminocarbonyl, C₁-C₆-Alkylaminothiocarbonyl, Di-C₁-C₆-Alkylaminothiocarbonyl, C₂-C₆-Alkenyl, C₂-C₆-Alkenyloxy, Benzyl, Benzyloxy, Aryl, Aryloxy, Hetaryl, Hetaryloxy oder C(=NOR⁶)-Aₙ-R⁷;
wobei
- A: für Sauerstoff, Schwefel oder Stickstoff steht und wobei der Stickstoff Wasserstoff oder C₁-C₆-Alkyl trägt;
- m: 0 oder 1;
- R⁶: Wasserstoff, C₁-C₆-Alkyl, C₂-C₆-Alkenyl oder C₂-C₆-Alkinyl bedeutet und
- R⁷: Wasserstoff, C₁-C₆-Alkyl, C₂-C₆-Alkenyl oder C₂-C₆-Alkinyl bedeutet,
sowie deren Salze.

Außerdem betrifft die Erfindung Verfahren und Zwischenprodukte zur Herstellung dieser Verbindungen sowie sie enthaltende Mittel zur Bekämpfung tierischer Schädlinge und Schadpilze.

Aus der Literatur sind Anilide als Fungizide bekannt (WO-A 93/15 046).

Der vorliegenden Erfindung lagen neue Verbindungen mit verbesserter Wirkung als Aufgabe zugrunde.

Demgemaß wurden die eingangs definierten Iminooximethylenanilide I gefunden. Außerdem wurden Verfahren und Zwischenprodukte zu ihrer Herstellung sowie sie enthaltende Mittel zur Bekämpfung von tierischen Schädlingen und Schadpilzen und ihre Verwendung in diesem Sinne gefunden.

Die Verbindungen I sind auf verschiedenen Wegen nach an sich in der Literatur bekannten Verfahren erhältlich.

Grundsätzlich ist es bei der Synthese der Verbindungen I unerheblich, ob zunächst die Gruppierung -N(OR)-COXR¹ oder die Gruppierung -CH₂ON=C(R³)-C(R⁴)=NOR⁵ aufgebaut wird.

Der Aufbau der Gruppierung -N(OR)-COXR¹ ist beispielsweise aus der eingangs zitierten Literatur bekannt. Im allgemeinen kann die Synthese ausgehend von Verbindungen der Formel XA erfolgen, 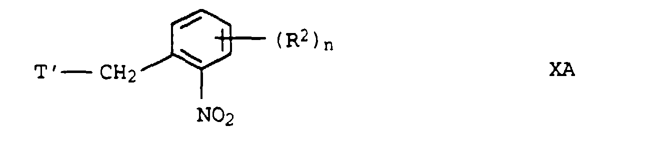 in der T' Wasserstoff oder eine Gruppe O=C(R⁴)-C(R³)=NO-, HON=C(R⁴)-C(R³)=NO- oder R⁵ON=C(R⁴)-C(R³)=NO- bedeutet

Verbindungen XB, in denen T' nicht Wasserstoff bedeutet, können gemäß den im folgenden in den Punkten 1 bis 6 beschriebenen Verfahren hergestellt werden.

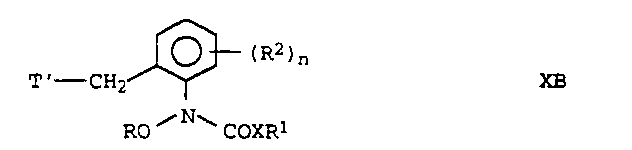

Die Art der Synthese der -CH₂ON=C(R³)-C(R⁴)=NOR⁵ Seitenkette richtet sich im wesentlichen nach der Art der Substituenten R³ und R⁴.
1. Für den Fall, daß R³ und R⁴ unabhängig voneinander für Wasserstoff, einen der angegebenen C-organischen Reste oder für Hydroxy oder Alkoxy stehen, geht man beim Aufbau der Gruppierung -CH₂ON=C(R³)-C(R⁴)=NOR⁵ im allgemeinen so vor, daß man ein Benzylderivat der Formel II mit einem Oxim der Formel III umsetzt. 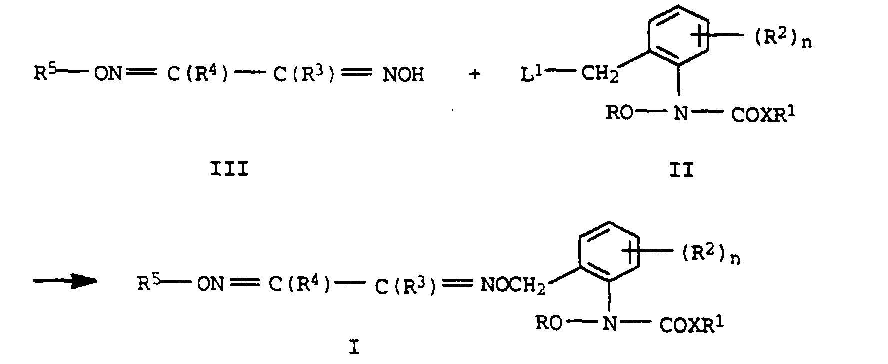
   L¹ in der Formel II steht für eine nukleophil austauschbare Abgangsgruppe, z.B. Halogen oder Sulfonatgruppen, vorzugsweise Chlor, Brom, Iod, Mesylat, Tosylat oder Triflat.
   Die Umsetzung erfolgt in an sich bekannter Weise in einem inerten organischen Lösungsmittel in Gegenwart einer Base, z.B. Natriumhydrid, Kaliumhydroxid, Kaliumcarbonat und Triethylamin gemäß den in Houben-Weyl, Bd. E 14b, S. 370f und Houben-Weyl, Bd. 10/1, S. 1189f beschriebenen Methoden.
   Das benötigte Oxim III erhält man durch Umsetzung eines entsprechenden Dioxims IV mit einem nukleophil substituierten Reagens VI 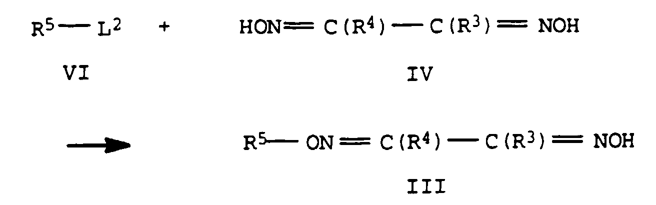
   L² in der Formel VI steht für eine nukleophil austauschbare Abgangsgruppe, z.B. Halogen oder Sulfonatgruppen, vorzugsweise Chlor, Brom, Iod, Mesylat, Tosylat oder Triflat.
   Die Umsetzung erfolgt in an sich bekannter Weise in einem inerten organischen Lösungsmittel in Gegenwart einer Base, z.B. Kaliumcarbonat, Kaliumhydroxid, Natriumhydrid, Pyridin und Triethylamin gemäß den in Houben-Weyl, Bd. E 14b, S. 307f, S. 370f und S. 385f; Houben-Weyl, Bd. 10/4, S. 55f, S. 180f und S. 217f; Houben-Weyl, Bd. E 5, S. 780f, beschriebenen Methoden.
1.1 Alternativ können die Verbindungen I auch dadurch erhalten werden, daß das Benzylderivat II zunächst mit dem Dioximderivat IV in ein entsprechendes Benzyloxim der Formel V umgesetzt wird, wobei V anschließend mit dem nukleophil substituierten Reagens VI zu I umgesetzt wird. 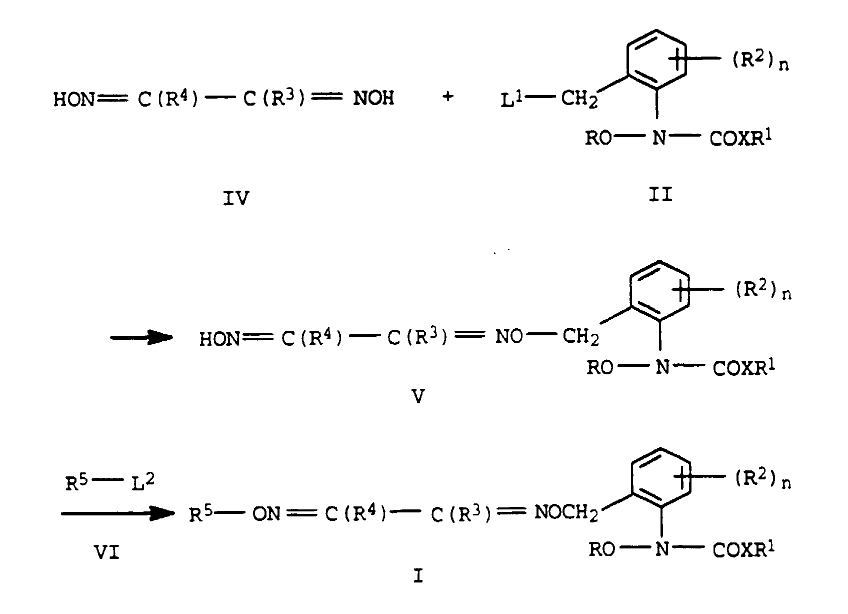
   Die Umsetzung erfolgt in an sich bekannter Weise in einem inerten organischen Lösungsmittel in Gegenwart einer Base z.B. Kaliumcarbonat, Kaliumhydroxid, Natriumhydrid, Pyridin und Triethylamin gemäß den in Houben-Weyl, Bd. 10/1, S. 1189f; Houben-Weyl, Bd. E 14b, S. 307f, S. 370f und S. 385f; Houben-Weyl, Bd. 10/4, S. 55f, S. 180f und S. 217f; Houben-Weyl, Bd. E 5, S. 780f, beschriebenen Methoden.
1.2 Analog ist es ebenfalls möglich, das benötigte Oxim der Formel III aus einem Ketooxim VII durch Umsetzung mit einem Hydroxylamin IXa oder seinem Salz IXb herzustellen. 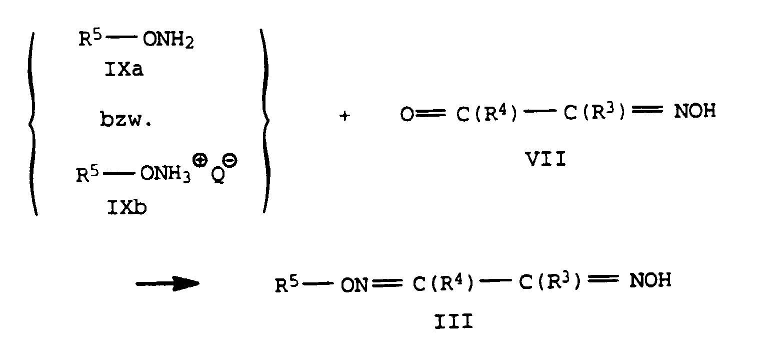
   Q^{⊖} in der Formel IXb steht für das Anion einer Säure, insbesondere einer anorganischen Saure, z.B. Halogenid wie Chlorid.
   Die Umsetzung erfolgt in an sich bekannter Weise in einem inerten organischen Lösungsmittel gemäß den in EP-A 513 580; Houben-Weyl, Bd. 10/4, S. 73f; Houben-Weyl, Bd. E 14b, S. 369f und S. 385f beschriebenen Methoden.
1.3 Alternativ können die Verbindungen I auch dadurch erhalten werden, daß das Benzylderivat II zunächst mit dem Ketooximderivat VII in ein entsprechendes Benzylketoxim der Formel VIII umgesetzt wird, wobei VIII anschließend mit dem Hydroxylamin IXa bzw. dessen Salz IXb zu I umgesetzt wird. 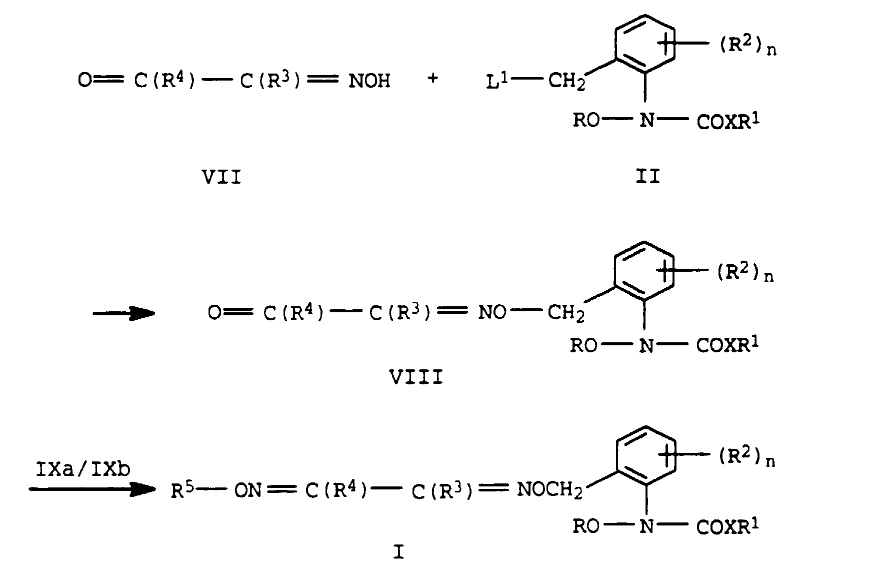
   Die Umsetzung erfolgt in an sich bekannter Weise in einem inerten organischen Lösungsmittel gemäß den in Houben-Weyl, Bd. E 14b, S. 369f; Houben-Weyl, Bd. 10/1, S. 1189f und Houben-Weyl, Bd. 10/4, S. 73f oder EP-A 513 580 beschriebenen Methoden.
1.4 Eine weitere Möglichkeit zur Herstellung der Verbindungen I ist die Umsetzung des Benzylderivats II mit N-Hydroxyphthalimid und nachfolgender Hydrazinolyse zum Benzylhydroxylamin IIa und die weitere Umsetzung von IIa mit einer Carbonylverbindung X. 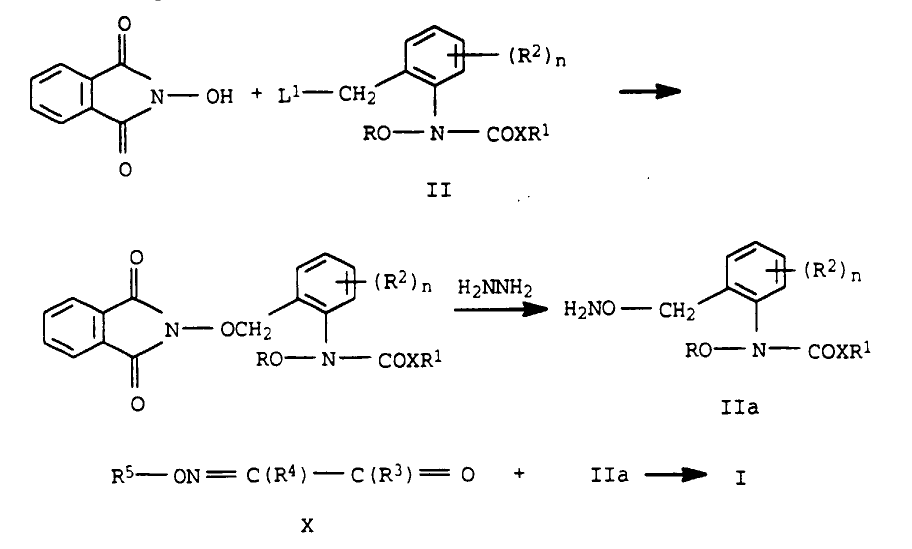
   Die Umsetzung erfolgt in an sich bekannter Weise in einem inerten organischen Lösungsmittel gemäß den in EP-A 463 488, DE-Anm. Nr. 42 28 867.3 beschriebenen Methoden.
   Die benötigte Carbonylverbindung X erhalt man durch Umsetzung eines entsprechenden Oximketons VIIa mit einem nukleophil substituierten Reagens VI 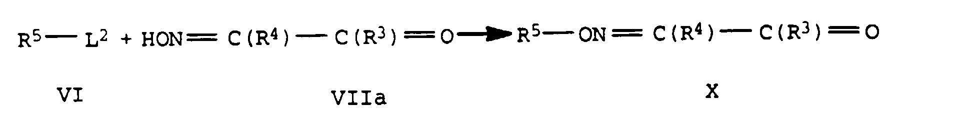 oder durch Umsetzung eines entsprechenden Diketons XI mit einem Hydroxylamin IXa oder dessen Salz IXb 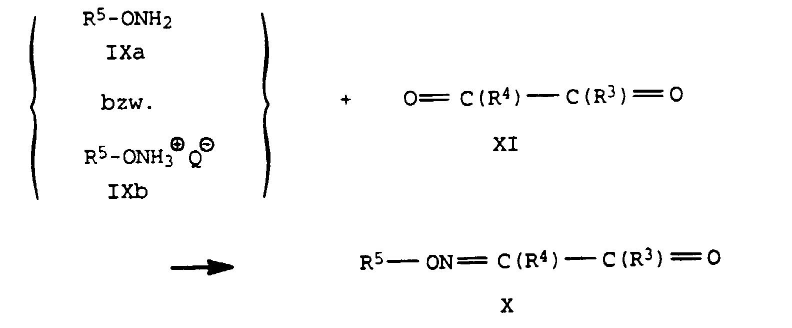
   Die Umsetzungen erfolgen in an sich bekannter Weise in einem inerten organischen Lösungsmittel gemäß den in EP-A 513 580, Houben-Weyl, Bd. 10/4, S. 55f, S. 73f, S. 180f und S. 217f; Houben-Weyl, Bd. E 14b, S. 307f und 369f, Houben-Weyl, Bd. E 5, S. 780f beschriebenen Methoden.
1.5 Entsprechend können die Verbindungen I auch dadurch erhalten werden, daß das Benzylhydroxylamin IIa zunächst mit dem Oximketon VIIa in das entsprechende Benzyloxim der Formel V umgesetzt wird, wobei V anschließend mit dem nukleophil substituierten Reagens VI wie vorstehend beschrieben zu I umgesetzt wird. 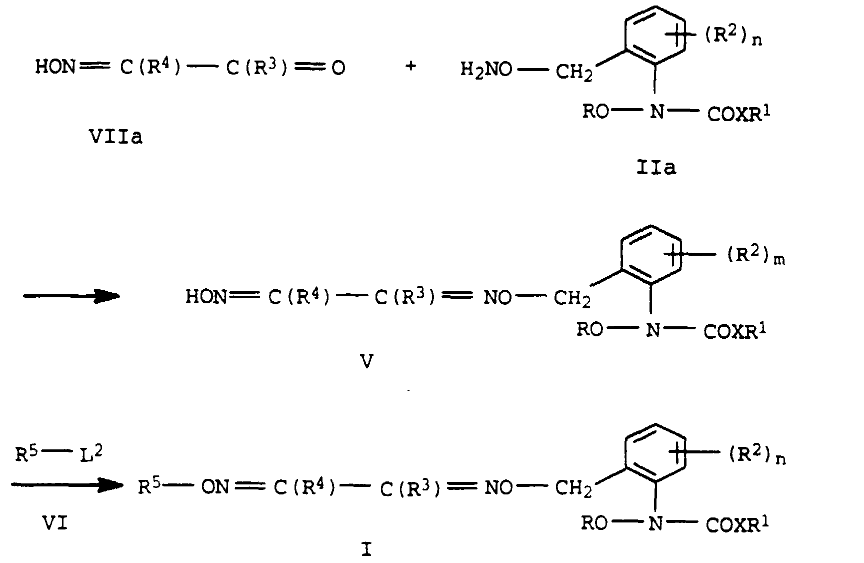
1.6 Analog können die Verbindungen I ebenfalls dadurch hergestellt werden, daß das Benzylhydroxylamin IIa zunächst mit dem Diketon der Formel XI in das Benzyloxim der Formel VIII überführt wird und VIII anschließend mit dem Hydroxylamin IXa oder dessen Salz IXb wie vorstehend beschrieben zu I umgesetzt wird. 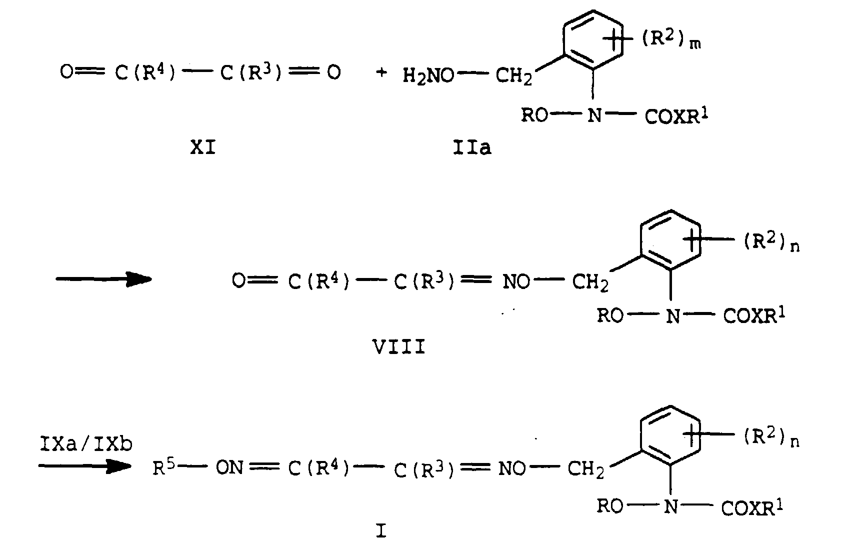
2. Verbindungen, in denen R³ und/oder R⁴ für ein Halogenatom stehen, erhält man aus den entsprechenden Vorstufen, in denen der betreffende Rest für eine Hydroxygruppe steht nach an sich bekannten Methoden (vgl. Houben-Weyl, Vol. E5, S. 631; J. Org. Chem. 36, 233 (1971); J. Org. Chem. 57, 3245 (1992)). Vorzugsweise werden die entsprechenden Umsetzungen zum Halogenderivat auf den Stufen I und VIII vorgenommen.
3. Verbindungen, in denen R³ und/oder R⁴ für eine Alkoxy- oder Alkylthiogruppe stehen, erhält man aus den entsprechenden Vorstufen, in denen der betreffende Rest für ein Halogenatom steht nach an sich bekannten Methoden (vgl. Houben-Weyl, Bd. E5, S. 826 f und 1280 f, J. Org. Chem. 36, 233 (1971), J. Org. Chem. 46, 3623 (1981)). Vorzugsweise werden die entsprechenden Umsetzungen der Halogenderivat auf den Stufen I und VIII vorgenommen.
4. Verbindungen, in denen R³ und/oder R⁴ für eine Alkoxygruppe stehen, erhält man auch aus den entsprechenden Vorstufen, in denen der betreffende Rest für eine Hydroxygruppe steht nach an sich bekannten Methoden (vgl. Houben-Weyl, Bd. E5, S. 826-829, Aust. J. Chem. 27, 1341-9 (1974)). Vorzugsweise werden die entsprechenden Umsetzungen zu den Alkoxyderivaten auf den Stufen I und VIII vorgenommen.
   Verbindungen der Formel I, in denen X für NR^{a} steht, können außerdem nach den folgenden Verfahren erhalten werden:
5. Beispielsweise erhält man derartige Verbindungen ausgehend von Zwischenprodukten der Formel X, 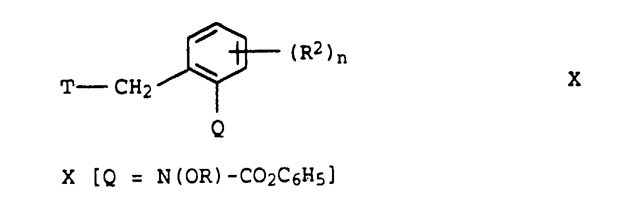 in denen Q für N(OR)-CO₂C₆H₅ steht und T O=C(R⁴)-C(R³)=NO-, HON=C (R⁴)-C(R³)=NO- oder R⁵ON=C (R⁴)-C(R³)=NO- bedeutet, durch Umsetzung mit einem Amin der Formel HNR^{a}R¹ (bzw. Ammoniak für den Fall, daß R^{a} und R¹ Wasserstoff bedeuten). 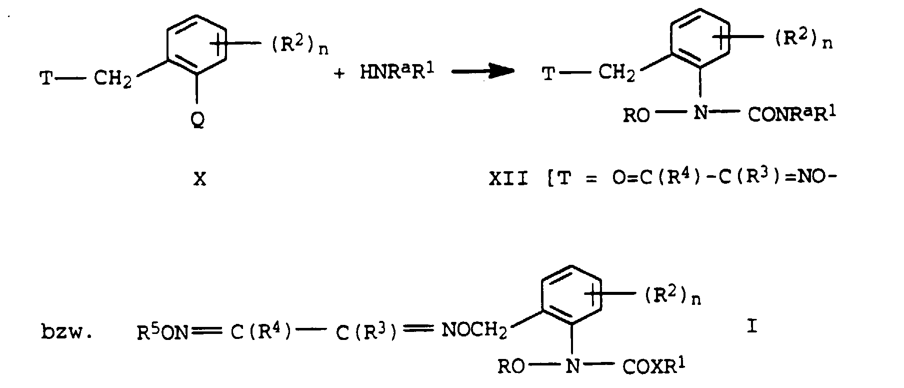
   Die Umwandlung der Seitenkette (für den Fall, daß T O=C(R⁴)-C(R³)=NO- oder HON=C (R⁴)-C(R³)=NO- bedeutet ≡ Formel XII) erfolgt nach den vorstehend beschriebenen Methoden.
6. Alternativ zu der in Punkt 5. beschriebenen Herstellungsmethode erhält man die Verbindungen I, in denen X für NR^{a} steht, auch gemäß dem folgenden Reaktionsschema ausgehend von Verbindungen I (oder deren Vorstufen), in denen XR¹ für eine C₁-C₄-Alkoxygruppe steht.

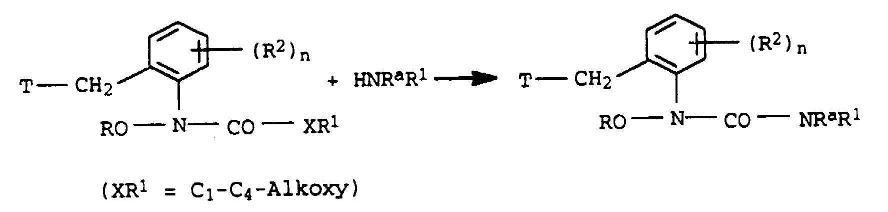

Die Verbindungen II sind bekannt (WO-A 93/15 046) oder können nach den dort beschriebenen Methoden hergestellt werden.

Die Verbindungen I können bei der Herstellung aufgrund ihrer C=N Doppelbindungen als E/Z-Isomerengemische anfallen, die z.B. durch Kristallisation oder Chromatographie in üblicher Weise in die Einzelverbindungen getrennt werden können.

Sofern bei der Synthese Isomerengemische anfallen, ist im allgemeinen jedoch eine Trennung nicht unbedingt erforderlich, da sich die einzelnen Isomere teilweise während der Aufbereitung für die Anwendung oder bei der Anwendung (z.B. unter Licht-, Säure- oder Baseneinwirkung) ineinander umwandeln können. Entsprechende Umwandlungen können auch nach der Anwendung, beispielsweise bei der Behandlung von Pflanzen in der behandelten Pflanze oder im zu bekämpfenden Schadpilz oder tierischen Schädling erfolgen.

In Bezug auf die -C(R³)=NOCH₂- Doppelbindung werden hinsichtlich ihrer Wirksamkeit die cis-Isomere der Verbindungen I bevorzugt (Konfiguration bezogen auf den Rest R³ im Verhältnis zur -OCH₂-Gruppe).

Die Verbindungen I können saure oder basische Zentren enthalten und dementsprechend Säureadditionsprodukte oder Basenadditionsprodukte oder Salze bilden.

Säuren für Säureadditionsprodukte sind u.a. Mineralsäuren (z.B. Halogenwasserstoffsäuren wie Chlorwasserstoff- und Bromwasserstoffsäure, Phosphorsäure, Schweferlsäure, Salpetersäure), organische Säuren (z.B. Ameisensäure, Essigsäure, Oxalsäure, Malonsäure, Milchsäure, Äpfelsäure, Bernseinsäure, Weinsäure, Zitronensäure, Salizylsäure, p-Toluolsulfonsäure, Dodecylbenzolsulfonsäure) oder andere protonenacide Verbindugnen (z.B. Saccharin).

Basen für Basenadditionsprodukte sind u.a. Oxide, Hydroxide, Carbonate oder Hydrogencarbonate von Alkalimetallen oder Erdalkaimetallen (z.B. Kalium- oder Natriumhydroxyd oder -carbonat) oder Ammoniumverbindungen (z.B. Ammoniumhydroxyd).

Bei der eingangs angegebenen Definitionen der Verbindungen I wurden Sammelbegriffe verwendet, die allgemein repräsentativ für die folgenden Gruppen stehen:
**Halogen:** Fluor, Chlor, Brom und Jod;
**Alkyl:** geradkettige oder verzweigte Alkylgruppen mit 1 bis 4, 6 oder 10 Kohlenstoffatomen, z.B. C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1- Ethyl-2-methylpropyl;
**Alkylamino:** eine Aminogruppe, welche eine geradkettige oder verzweigte Alkylgruppe mit 1 bis 6 Kohlenstoffatomen wie vorstehend genannt trägt;
**Dialkylamino:** eine Aminogruppe, welche zwei voneinander unabhängige, geradkettige oder verzweigte Alkylgruppen mit jeweils 1 bis 6 Kohlenstoffatomen wie vorstehend genannt, tragt;
**Alkylcarbonyl:** geradkettige oder verzweigte Alkylgruppen mit 1 bis 10 Kohlenstoffatomen, welche über eine Carbonylgruppe (-CO-) an das Gerüst gebunden sind;
**Alkylsulfonyl:** geradkettige oder verzweigte Alkylgruppen mit 1 bis 6 oder 10 Kohlenstoffatomen, welche über eine Sulfonylgruppe (-SO₂-) an das Gerüst gebunden sind;
**Alkylsulfoxyl:** geradkettige oder verzweigte Alkylgruppen mit 1 bis 6 Kohlenstoffatomen, welche über eine Sulfoxylgruppe (-S(=O)-) an das Gerüst gebunden sind;
**Alkylaminocarbonyl:** Alkylaminogruppen mit 1 bis 6 Kohlenstoffatomen wie vorstehend genannt, welche über eine Carbonylgruppe (-CO-) an das Gerüst gebunden sind;
**Dialkylaminocarbonyl:** Dialkylaminogruppen mit jeweils 1 bis 6 Kohlenstoffatomen pro Alkylrest wie vorstehend genannt, welche über eine Carbonylgruppe (-CO-) an das Gerüst gebunden sind;
**Alkylaminothiocarbonyl:** Alkylaminogruppen mit 1 bis 6 Kohlenstoffatomen wie vorstehend genannt, welche über eine Thiocarbonylgruppe (-CS-) an das Gerüst gebunden sind;
**Dialkylaminothiocarbonyl:** Dialkylaminogruppen mit jeweils 1 bis 6 Kohlenstoffatomen pro Alkylrest wie vorstehend genannt, welche über eine Thiocarbonylgruppe (-CS-) an das Gerüst gebunden sind;
**Halogenalkyl:** geradkettige oder verzweigte Alkylgruppen mit 1 bis 6 Kohlenstoffatomen, wobei in diesen Gruppen teilweise oder vollständig die Wasserstoffatome durch Halogenatome wie vorstehend genannt ersetzt sein können, z.B. C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
**Alkoxy:** geradkettige oder verzweigte Alkylgruppen mit 1 bis 4 oder 6 Kohlenstoffatomen wie vorstehend genannt, welche über ein Sauerstoffatom (-O-) an das Gerüst gebunden sind, z.B. C₁-C₆-Alkoxy wie Methyloxy, Ethyloxy, Propyloxy, 1-Methylethyloxy, Butyloxy, 1-Methyl-propyloxy, 2-Methylpropyloxy, 1,1-Dimethylethyloxy, Pentyloxy, 1-Methylbutyloxy, 2-Methylbutyloxy, 3-Methylbutyloxy, 2,2-Di-methylpropyloxy, 1-Ethylpropyloxy, Hexyloxy, 1,1-Dimethylpropyloxy, 1,2-Dimethylpropyloxy, 1-Methylpentyloxy, 2-Methylpentyloxy, 3-Methylpentyloxy, 4-Methylpentyloxy, 1,1-Dimethylbutyloxy, 1,2-Dimethylbutyloxy, 1,3-Dimethylbutyloxy, 2,2-Dimethylbutyloxy, 2,3-Dimethylbutyloxy, 3,3-Dimethylbutyloxy, 1-Ethyl-butyloxy, 2-Ethylbutyloxy, 1,1,2-Trimethylpropyloxy, 1,2,2-Trimethylpropyloxy, 1-Ethyl-1-methylpropyloxy und 1-Ethyl-2-methylpropyloxy;
**Alkoxycarbonyl:** geradkettige oder verzweigte Alkylgruppen mit 1 bis 6 Kohlenstoffatomen, welche über eine Oxycarbonylgruppe (-OC(=O)-) an das Gerüst gebunden sind;
**Halogenalkoxy:** geradkettige oder verzweigte Alkylgruppen mit 1 bis 6 Kohlenstoffatomen, wobei in diesen Gruppen teilweise oder vollstandig die Wasserstoffatome durch Halogenatome wie vorstehend genannt ersetzt sein können, und wobei diese Gruppen über ein Sauerstoffatom an das Gerüst gebunden sind;
**Alkylthio:** geradkettige oder verzweigte Alkylgruppen mit 1 bis 4 oder 6 Kohlenstoffatomen wie vorstehend genannt, welche über ein Schwefelatom (-S-) an das Gerüst gebunden sind, z.B. C₁-C₆-Alkylthio wie Methylthio, Ethylthio, Propylthio, 1-Methylethylthio, Butylthio, 1-Methylpropylthio, 2-Methylpropylthio, 1,1-Dimethylethylthio, Pentylthio, 1-Methylbutylthio, 2-Methylbutylthio, 3-Methylbutylthio, 2,2-Di-methylpropylthio, 1-Ethylpropylthio, Hexylthio, 1,1-Dimethylpropylthio, 1,2-Dimethylpropylthio, 1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio und l-Ethyl-2-methylpropylthio;
**Cycloalkyl:** monocyclische Alkylgruppen mit 3 bis 6 Kohlenstoffringgliedern, z.B. Cyclopropyl, Cyclobutyl, Cyclopentyl und Cyclohexyl;
**Alkynyl:** geradkettige oder verzweigte Alkenylgruppen mit 2 bis 6 oder 10 Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-l-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Di-methyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-l-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1- Ethyl-1-methyl-2-propenyl, l-Ethyl-2-methyl-l-propenyl und 1-Ethyl-2-methyl-2-propenyl;
**Alkenyloxy:** geradkettige oder verzweigte Alkenylgruppen mit 2 bis 6 Kohlenstoffatomen und einer Doppelbindung in einer beliebigen Position, welche über ein Sauerstoffatom (-O-) an das Gerüst gebunden sind;
**Alkenylthio bzw. Alkenylamino:** geradkettige oder verzweigte Alkenylgruppen mit 2 bis 6 Kohlenstoffatomen und einer Doppelbindung in einer beliebigen Position, welche (Alkenylthio) über ein Schwefelatom bzw. (Alkenylamino) ein Stickstoffatom an das Gerüst gebunden sind.
**Alkenylcarbonyl:** geradkettige oder verzweigte Alkenylgruppen mit 2 bis 10 Kohlenstoffatomen und einer Doppelbindung in einer beliebigen Position, welche über eine Carbonylgruppe (-CO-) an das Gerüst gebunden sind;
**Alkinyl:** geradkettige oder verzweigte Alkinylgruppen mit 3 bis 10 Kohlenstoffatomen und einer Dreifachbindung in einer beliebigen Position, z.B. C₃-C₆-Alkinyl wie 2-Propinyl, 2-Butinyl, 3-Butinyl, 1-Methyl-2-propinyl, 2-Pentinyl, 3-Pentinyl, 4-Pentinyl, 1-Methyl-2-butinyl, 1-Methyl-3-butinyl, 2-Methyl-3-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, l,l-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und l-Ethyl-l-methyl-2-propinyl;
**Alkinyloxy bzw. Alkinylthio und Alkinylamino:** geradkettige oder verzweigte Alkinylgruppen mit 2 bis 6 Kohlenstoffatomen und einer Dreifachbindung in einer beliebigen Position, welche (Alkinyloxy) über ein Sauerstoffatom bzw. (Alkinylthio) über ein Schwefelatom oder (Alkinylamino) über ein Stickstoffatom an das Gerüst gebunden sind.
**Alkinylcarbonyl:** geradkettige oder verzweigte Alkinylgruppen mit 3 bis 10 Kohlenstoffatomen und einer Dreifachbindung in einer beliebigen Position, welche über eine Carbonylgruppe (-CO-) an das Gerüst gebunden sind;
**Cycloalkenyl bzw. Cycloalkenyloxy, Cycloalkenylthio und Cycloalkenylamino:** monocyclische Alkenylgruppen mit 3 bis 6 Kohlenstoffringgliedern, welche direkt bzw. (Cycloalkenyloxy) über ein Sauerstoffatom oder (Cycloalkenylthio) ein Schwefelatom oder Cycloalkenylamino) über ein Stickstoffatom an das Gerüst gebunden sind, z.B. Cyclopropenyl, Cyclobutenyl, Cyclopentenyl oder Cyclohexenyl.
**Cycloalkoxy bzw. Cycloalkylthio und Cycloalkylamino:** monocyclische Alkenylgruppen mit 3 bis 6 Kohlenstoffringgliedern, welche (Cycloalkyloxy) über ein Sauerstoffatom oder (Cycloalkylthio) ein Schwefelatom oder (Cycloalkylamino) über ein Stickstoffatom an das Gerüst gebunden sind, z.B. Cyclopropyl, Cyclobutyl, Cyclopentyl oder Cyclohexyl;
**Heterocyclyl bzw. Heterocyclyloxy, Heterocyclylthio und Heterocyclylamino:** drei- bis sechsgliedrige, gesättigte oder partiell ungesättigte mono- oder polycyclische Heterocyclen, die ein bis drei Hereroatome ausgewählt aus einer Gruppe bestehend aus Sauerstoff, Stickstoff und Schwefel enthalten, und welche direkt bzw. (Heterocyclyloxy) über ein Sauerstoffatom oder (Heterocyclylthio) über ein Schwefelatom oder (Heterocyclylamino) über ein Stickstoffatom an das Gerüst gebunden sind, wie z.B. 2-Tetrahydrofuranyl, Oxiranyl, 3-Tetrahydrofuranyl, 2-Tetrahydrothienyl, 3-Tetrahydrothienyl, 2-Pyrrolidinyl, 3-Pyrrolidinyl, 3-Isoxazoldinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,3-Dihydro-fur-4-yl, 2,3-Dihydro-fur-5-yl, 2,5-Dihydro-fur-2-yl, 2,5-Dihydro-fur-3-yl, 2,3-Dihydrothien-2-yl, 2,3-Dihydrothien-3-yl, 2,3-Dihydrothien-4-yl, 2,3-Dihydrothien-5-yl, 2,5-Dihydrothien-2-yl, 2,5-Dihydrothien-3-yl, 2,3-Dihydropyrrol-2-yl, 2,3-Dihydropyrrol-3-yl, 2,3-Dihydropyrrol-4-yl, 2,3-Dihydropyrrol-5-yl, 2,5-Dihydropyrrol-2-yl, 2,5-Dihydropyrrol-3-yl, 2,3-Dihydroisoxazol-3-yl, 2,3-Dihydroisoxazol-4-yl, 2,3-Dihydroisoxazol-5-yl, 4,5-Dihydroisoxazol-3-yl, 4,5-Dihydroisoxazol-4-yl, 4,5-Dihydroisoxazol-5-yl, 2,5-Dihydroisothiazol-3-yl, 2,5-Dihydroisothiazol-4-yl, 2,5-Dihydroisothiazol-5-yl, 2,3-Dihydroisopyrazol-3-yl, 2,3-Dihydroisopyrazol-4-yl, 2,3-Dihydroisopyrazol-5-yl, 4,5-Dihydroisopyrazol-3-yl, 4,5-Dihydroisopyrazol-4-yl, 4,5-Dihydroisopyrazol-5-yl, 2,5-Dihydroisopyrazol-3-yl, 2,5-Dihydroisopyrazol-4-yl, 2,5-Dihydroisopyrazol-5-yl, 2,3-Dihydrooxazol-3-yl, 2,3-Dihydrooxazol-4-yl, 2,3-Dihydrooxazol-5-yl, 4,5-Dihydrooxazol-3-yl, 4,5-Dihydrooxazol-4-yl, 4,5-Dihydrooxazol-5-yl, 2,5-Dihydrooxazol-3-yl, 2,5-Dihydrooxazol-4-yl, 2,5-Dihydrooxazol-5-yl, 2,3-Dihydrothiazol-2-yl, 2,3-Dihydrothiazol-4-yl, 2,3-Dihydrothiazol-5-yl, 4,5-Dihydrothiazol-2-yl, 4,5-Dihydrothiazol-4-yl, 4,5-Dihydrothiazol-5-yl, 2,5-Dihydrothiazol-2-yl, 2,5-Dihydrothiazol-4-yl, 2,5-Dihydrothiazol-5-yl, 2,3-Dihydroimidazol-2-yl, 2,3-Dihydroimidazol-4-yl, 2,3-Dihydroimidazol-5-yl, 4,5-Dihydroimidazol-2-yl, 4,5-Dihydroimidazol-4-yl, 4,5-Dihydroimidazol-5-yl, 2,5-Dihydroimidazol-2-yl, 2,5-Dihydroimidazol-4-yl, 2,5-Dihydroimidazol-5-yl, 2-Morpholinyl, 3-Morpholinyl, 2-Piperidinyl, 3-Piperidinyl, 4-Piperidinyl, 3-Tetrahydropyridazinyl, 4-Tetrahydropyridazinyl, 2-Tetrahydropyrimidinyl, 4-Tetrahydropyrimidinyl, 5-Tetrahydropyrimidinyl, 2-Tetrahydropyrazinyl, 1,3,5-Tetrahydrotriazin-2-yl, 1,2,4-Tetrahydrotriazin-3-yl, 1,3-Dihydrooxazin-2-yl, 1,3-Dithian-2-yl, 2-Tetrahydropyranyl, 1,3-Dioxolan-2-yl, 3,4,5,6-Tetrahydropyridin-2-yl, 4H-1;3-Thiazin-2-yl, 4H-3,1-Benzothiazin-2-yl, 1,1-Dioxo-2,3,4,5-tetrahydrothien-2-yl, 2H-1,4-Benzothiazin-3-yl, 2H-1,4-Benzoxazin-3-yl, 1,3-Dihydrooxazin-2-yl, 1,3-Dithian-2-yl,
**Aryl bzw. Aryloxy, Arylthio, Arylcarbonyl und Arylsulfonyl:** aromatische mono- oder polycyclische Kohlenwasserstoffreste welche direkt bzw. (Aryloxy) über ein Sauerstoffatom (-O-) oder (Arylthio) ein Schwefelatom (-S-), (Arylcarbonyl) über eine Carbonylgruppe (-CO-) oder (Arylsulfonyl) über eine Sulfonylgruppe (-SO₂-) an das Gerüst gebunden sind, z.B. Phenyl, Naphthyl und Phenanthrenyl bzw. Phenyloxy, Naphthyloxy und Phenanthrenyloxy und die entsprechenden Carbonyl- und Sulfonylreste;
**Arylamino:** aromatische mono- oder polycyclische Kohlenwasserstoffreste, welche über ein Stickstoffatom an das Gerüst gebunden sind.

**Hetaryl bzw. Hetaryloxy, Hetarylthio, Hetarylcarbonyl und Hetarylsulfonyl:** aromatische mono- oder polycyclische Reste welche neben Kohlenstoffringgliedern zusätzlich ein bis vier Stickstoffatome, oder ein bis drei Stickstoffatome und ein Sauerstoff- oder ein Schwefelatom, oder ein Sauerstoff- oder ein Schwefelatom enthalten können und welche direkt bzw. (Hetaryloxy) über ein Sauerstoffatom (-O-) oder (Hetarylthio) ein Schwefelatom (-S-), (Hetarylcarbonyl) über eine Carbonylgruppe (-CO-) oder (Hetarylsulfonyl) über eine Sulfonylgruppe (-SO₂-) an das Gerüst gebunden sind, z.B.
- 5-gliedriges Heteroaryl, enthaltend ein bis drei Stickstoff-atome: 5-Ring Heteroarylgruppen, welche neben Kohlenstoffatomen ein bis drei Stickstoffatome als Ringglieder enthalten können, z.B. 2-Pyrrolyl, 3-Pyrrolyl, 3-Pyrazolyl, 4-Pyrazolyl, 5-Pyrazolyl, 2-Imidazolyl, 4-Imidazolyl, 1,2,4- Triazol-3-yl, 1,2,3-Triazolyl und 1,3,4-Triazol-2-yl;
- 5-gliedriges Heteroaryl, enthaltend ein bis vier Stickstoffatome oder ein bis drei Stickstoffatome und ein Schwefeloder Sauerstoffatom oder ein Sauerstoff oder ein Schwefelatom: 5-Ring Heteroarylgruppen, welche neben Kohlenstoffatomen ein bis vier Stickstoffatome oder ein bis drei Stickstoffatome und ein Schwefel- oder Sauerstoffatom, oder ein Sauerstoff- oder Schwefelatom als Ringglieder enthalten können, z.B. 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4- Thiadiazol -3-yl, 1,2,4-Thiadiazol-5-yl, 1,2,4-Triazol-3-yl, 1,3,4-Oxadiazol-2-yl, 1,3,4-Thiadiazol-2-yl, 1,3,4- Triazol-2-yl;
- benzokondensiertes 5-gliedriges Heteroaryl, enthaltend ein bis drei Stickstoffatome oder ein Stickstoffatom und/oder ein Sauerstoff- oder Schwefelatom: 5-Ring Heteroarylgruppen, welche neben Kohlenstoffatomen ein bis vier Stickstoffatome, oder ein bis drei Stickstoffatome und ein Schwefel- oder Sauerstoffatom oder ein Sauerstoff- oder ein Schwefelatom als Ringglieder enthalten können, und in welchen zwei benachbarte Kohlenstoffringglieder oder ein Stickstoff- und ein benachbartes Kohlenstoffringglied durch eine Buta-1,3-dien-1,4-diylgruppe verbrückt sein können;
- über Stickstoff gebundenes 5-gliedriges Heteroaryl, enthaltend ein bis vier Stickstoffatome, oder über Stickstoff gebundenes benzokondensiertes 5-gliedriges Heteroaryl, enthaltend ein bis drei Stickstoffatome: 5-Ring Heteroarylgruppen, welche neben Kohlenstoffatomen ein bis vier Stickstoffatome bzw. ein bis drei Stickstoffatome als Ringglieder enthalten können, und in welchen zwei benachbarte Kohlenstoffringglieder oder ein Stickstoff- und ein benachbartes Kohlenstoffringglied durch eine Buta-1,3-dien- 1,4-diylgruppe verbrückt sein können, wobei diese Ringe über eines der Stickstoffringglieder an das Gerüst gebunden sind;
- 6-gliedriges Heteroaryl, enthaltend ein bis drei bzw. ein bis vier Stickstoffatome: 6-Ring Heteroarylgruppen, welche neben Kohlenstoffatomen ein bis drei bzw. ein bis vier Stickstoffatome als Ringglieder enthalten können, z.B. 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl und 1,2,4, 5-Tetrazin-3-yl;
- benzokondensiertes 6-gliedriges Heteroaryl, enthaltend ein bis vier Stickstoffatome: 6-Ring Heteroarylgruppen in welchen zwei benachbarte Kohlenstoffringglieder durch eine Buta-1,3-dien-1,4-diylgruppe verbrückt sein können, z.B. Chinolin, Isochinolin, Chinazolin und Chinoxalin,
bzw. die entsprechenden Oxy-, Thio-, Carbonyl- oder Sulfonylgruppen.

**Hetarylamino:** aromatische mono- oder polycyclische Reste, welche neben Kohlenstoffringgliedern zusätzlich ein bis vier Stickstoffatome oder ein bis drei Stickstoffatome und ein Sauerstoff- oder ein Schwefelatom enthalten können und welche über ein Stickstoffatom an das Gerüst gebunden sind.

Die Angabe "partiell oder vollständig halogeniert" soll zum Ausdruck bringen, daß in den derart charakterisierten Gruppen die Wasserstoffatome zum Teil oder vollständig durch gleiche oder verschiedene Halogenatome wie vorstehend genannt ersetzt sein können.

Im Hinblick auf ihre biologische Wirkung sind Verbindungen der Formel I bevorzugt, in denen n für 0 oder 1, insbesondere für 0, steht.

Daneben werden Verbindungen I bevorzugt, in denen R für Wasserstoff, C₁-C₄-Alkyl oder C₁-C₂-Halogenalkyl steht.

Außerdem werden Verbindungen I bevorzugt, in denen R¹X für Methyl, Ethyl, Methoxy oder Methylamino steht.

Gleichermaßen werden Verbindungen I bevorzugt, in denen R³ für Wasserstoff, Hydroxy, Cyano, Cyclopropyl, Chlor, Methyl, Ethyl, 1-Methylethyl, Trifluormethyl, Methoxy, Methylthio oder Phenyl steht.

Außerdem werden Verbindungen I bevorzugt, in denen R³ für Methyl steht.

Des weiteren werden Verbindungen I bevorzugt, in denen R³ für Methoxy steht.

Außerdem werden Verbindungen I bevorzugt, in denen R³ für Hydroxy steht.

Des weiteren werden Verbindungen I bevorzugt, in denen R³ für Chlor steht.

Des weiteren werden Verbindungen I bevorzugt, in denen R⁴ für Wasserstoff, Hydroxy, Cyclopropyl, Chlor, Methyl, Ethyl, iso-Propyl, n-Butyl, iso-Butyl, tert.-Butyl, Methoxy oder Methylthio steht.

Außerdem werden Verbindungen I bevorzugt, in denen R⁴ für Methyl steht.

Des weiteren werden Verbindungen I bevorzugt, in denen R⁴ für Methoxy steht.

Außerdem werden Verbindungen I bevorzugt, in denen R⁴ für Hydroxy steht.

Des weiteren werden Verbindungen I bevorzugt, in denen R⁴ für Ethyl steht.

Außerdem werden Verbindungen I bevorzugt, in denen R⁴ für Iso-Propyl steht.

Außerdem werden Verbindungen I bevorzugt, in denen R⁴ für Cyclopropyl steht.

Des weiteren werden Verbindungen I bevorzugt, in denen R⁴ für ggf. subst. Aryl oder Hetaryl steht.

Des weiteren werden Verbindungen I bevorzugt, in denen R⁴ für ggf. subst. Pyridyl, Pyrimidyl, Pyrazinyl, Pyridazinyl oder Triazinyl steht.

Des weiteren werden Verbindungen I bevorzugt, in denen R⁴ für ggf. subst. Furyl, Thienyl oder Pyrrolyl steht.
Des weiteren werden Verbindungen I bevorzugt, in denen R⁴ für ggf. subst. Oxazolyl, Thiazolyl, Isoxazolyl, Isothiazolyl, Pyrazolyl oder Imidazolyl steht.

Des weiteren werden Verbindungen I bevorzugt, in denen R⁴ für ggf. subst. Oxadiazolyl, Thiadiazolyl oder Triazolyl steht.

Außerdem werden Verbindungen I bevorzugt, in denen R⁴ für Phenyl steht, welches unsubstituiert ist oder ein oder zwei der folgenden Gruppen trägt: Nitro, Cyano, Hydroxy, Amino, Aminocarbonyl, Aminothiocarbonyl, Halogen, C₁-C₄-Alkyl, C₁-C₄-Halogenalkyl, C₁-C₄-Alkoxy, C₁-C₄-Halogenalkoxy, C₁-C₄-Alkylamino, Di-C₁-C₄-Alkylamino, C₁-C₄-Alkylsulfonyl, C₁-C₄-Alkoxycarbonyl, C₁-C₄-Alkylaminocarbonyl or Di-C₁-C₄-Alkylaminocarbonyl.

Außerdem werden Verbindungen I bevorzugt, in denen R⁵ für Wasserstoff, C₁-C₆-Alkyl, Arylalkyl, Hetarylalkyl, Aryloxyalkyl, Hetaryloxyalkyl, Aryl oder Hetaryl steht.

Des weiteren werden Verbindungen I bevorzugt, in denen R⁵ für C₁-C₆-Alkyl steht.

Außerdem werden Verbindungen I bevorzugt, in denen R⁵ für Methyl oder Ethyl steht.

Des weiteren werden Verbindungen I bevorzugt, in denen R⁵ für Arylalkyl oder Hetarylalkyl steht.

Außerdem werden Verbindungen I bevorzugt, in denen R⁵ für Aryloxyalkyl oder Hetaryloxyalkyl steht.

Außerdem werden Verbindungen I bevorzugt, in denen R⁵ für Aryl oder Hetaryl steht.

Die Verbindungen I eignen sich als Fungizide.

Die Verbindungen I zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Speziell eignen sie sich zur Bekämpfung folgender Pflanzenkrankheiten: Erysiphe graminis (echter Mehltau) in Getreide, Erysiphe cichoracearum und Sphaerotheca fuliginea an Kürbisgewächsen, Podosphaera leucotricha an Äpfeln, Uncinula necator an Reben, Puccinia-Arten an Getreide, Rhizoctonia-Arten an Baumwolle und Rasen, Ustilago-Arten an Getreide und Zuckerrohr, Venturia inaequalis (Schorf) an Äpfeln, Helminthosporium-Arten an Getreide, Septoria nodorum an Weizen, Botrytis cinerea (Grauschimmel) an Erdbeeren, Reben, Cercospora arachidicola an Erdnüssen, Pseudocercosporella herpotrichoides an Weizen, Gerste, Pyricularia oryzae an Reis, Phytophthora infestans an Kartoffeln und Tomaten, Fusarium- und Verticillium-Arten an verschiedenen Pflanzen, Plasmopara viticola an Reben, Alternaria-Arten an Gemüse und Obst.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung erfolgt vor oder nach der Infektion der Materialien, Pflanzen oder Samen durch die Pilze.

Sie können in die üblichen Formulierungen überführt werden, wie Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung des ortho-substituierten Benzylesters einer Cyclopropancarbonsäure gewährleisten. Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie LigninSulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,01 und 2,0 kg Wirkstoff pro ha.

Bei der Saatgutbehandlung werden im allgemeinen Wirkstoffmengen von 0,001 bis 0,1 g, vorzugsweise 0,01 bis 0,05 g je Kilogramm Saatgut benötigt.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zink-ethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'-propylenbis-dithiocarbamat), N,N'-Polypropylen-bis-(thiocarbamoyl)disulfid;
Nitroderivate, wie Dinitro-(1-methylheptyl)-phenylcrotonat, 2-sec-Butyl-4,6-dinitrophenyl-3,3-dimethylacrylat, 2-sec-Butyl-4,6-dinitrophenyl-isopropylcarbonat, 5-Nitro-isophthalsäure-di-isopropylester;
heterocyclische Substanzen, wie 2-Heptadecyl-2-imidazolin-acetat, 2,4-Dichlor-6-(o-chloranilino)-s-triazin, O,O-Diethyl-phthalimidophosphonothioat, 5-Amino-1-[bis-(dimethylamino)-phosphinyl]-3-phenyl-1,2,4- triazol, 2,3-Dicyano-1,4-dithioanthrachinon, 2-Thio-1,3-dithiolo[4,5-b]chinoxalin, 1-(Butylcarbamoyl)-2-benzimidazol-carbaminsäuremethylester, 2-Methoxycarbonylamino-benzimidazol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthiophthalimid,
N-Dichlorfluormethylthio-N',N'-dimethyl-N-phenyl-schwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methyl-furan-3-carbonsäureanilid, 2,5-Dimethyl-furan-3-carbonsäureanilid, 2,4,5-Trimethyl-furan-3-carbonsaureanilid, 2,5-Dimethyl-furan-3-carbonsäurecyclohexylamid, N-Cyclohexyl-N-methoxy-2,5-dimethyl-furan3-carbonsäureamid, 2-Methyl-benzoesäure-anilid, 2-Iod-benzoesäure-anilid, N-Formyl-N-morpholin-2,2,2-trichlorethylacetal, Piperazin-1,4-diylbis-(1-(2,2,2-trichlor-ethyl)-formamid, 1-(3,4-Dichloranilino)-1-formylamino-2,2,2-trichlorethan, 2,6-Dimethyl-N-tridecyl-morpholin bzw. dessen Salze, 2,6-Dimethyl-N-cyclododecyl-morpholin bzw. dessen Salze, N-[3-(p-tert.-Butylphenyl)-2-methylpropyl]-cis-2,6-dimethylmorpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1H-1,2,4-triazol, 1-[2-(2,4-Dichlorphenyl)-4-n-propyl-1,3-dioxolan-2-yl-ethyl]-1H-1,2,4-triazol, N-(n-Propyl)-N-(2,4,6-trichlorphenoxyethyl)-N'-imidazol-yl-harnstoff, 1-(4-Chlorphenoxy)-3,3-dimethyl-1-(1H-l,2,4-triazol-l-yl)-2-butanon, 1-(4-Chlorphenoxy)-3,3-dimethyl-1-(1H-l,2,4-triazol-1-yl)-2-butanol, α-(2-Chlorphenyl)-α-(4-chlorphenyl)-5-pyrimidin-methanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)-benzol, 1,2-Bis-(3-methoxycarbonyl-2-thioureido)-benzol, sowie verschiedene Fungizide, wie Dodecylguanidinacetat, 3-[3-(3,5-Dimethyl-2-oxycyclohexyl)-2-hydroxyethyl]-glutarimid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl)-alaninmethyl- ester, N-(2,6-Dimethylphenyl)-N-chloracetyl-D,L-2-amino-butyrolacton, DL-N- (2,6-Dimethylphenyl)-N- (phenylacetyl)-alaninmethylester, 5-Methyl-5-vinyl-3-(3,5-dichlorphenyl)-2,4-dioxo-1,3-oxazolidin, 3-[3,5-Dichlorphenyl(-5-methyl-5-methoxymethyl]-1,3-oxazolidin- 2,4-dion, 3-(3,5-Dichlorphenyl)-l-isopropylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, 1-[2-(2,4-Dichlorphenyl)-pentyl]-1H-1,2,4-triazol, 2,4-Difluor-α-(1H-l,2,4-triazolyl-l-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-1H-1,2,4-triazol.

Die Verbindungen der Formel I sind außerdem geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grandiosella, Earias insulana, Elasmopalpus lignosellus, Eupoecilia ambiguella, Evetria bouliana, Feltia subterranea, Galleria mellonella, Grapholitha funebrana, Grapholitha molesta, Heliothis armigera, Heliothis virescens, Heliothis zea, Hellula undalis, Hibernia defoliaria, Hyphantria cunea, Hyponomeuta malinellus, Keiferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flammea, Pectinophora gossypiella, Peridroma saucia, Phalera bucephala, Phthorimaea operculella, Phyllocnistis citrella, Pieris brassicae, Plathypena scabra, Plutella xylostella, Pseudoplusia includens, Rhyacionia frustrana, Scrobipalpula absoluta, Sitotroga cerealella, Sparganothis pilleriana, Spodoptera frugiperda, Spodoptera littoralis, Spodoptera litura, Thaumatopoea pityocampa, Tortrix viridana, Trichoplusia ni, Zeiraphera canadensis.

Aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrhynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Oulema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus, Sitophilus granaria.

Aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossina morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea, Tipula paludosa.

Aus der Ordnung der Thripse (Thysanoptera) beispielsweise Frankliniella fusca, Frankliniella occidentalis, Frankliniella tritici, Scirtothrips citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euschistus impictiventris, Leptoglossus phyllopus, Lygus lineolaris, Lygus pratensis, Nezara viridula, Piesma quadrata, Solubea insularis, Thyanta perditor.

Aus der Ordnung der Pflanzensauger (Homoptera) beispielsweise Acyrthosiphon onobrychis, Adelges laricis, Aphidula nasturtii, Aphis fabae, Aphis pomi, Aphis sambuci, Brachycaudus cardui, Brevicoryne brassicae, Cerosipha gossypii, Dreyfusia nordmannianae, Dreyfusia piceae, Dysaphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappaphis mala, Sappaphis mali, Schizaphis graminum, Schizoneura lanuginosa, Trialeurodes vaporariorum, Viteus vitifolii.

Aus der Ordnung der Termiten (Isoptera) beispielsweise Calotermes flavicollis, Leucotermes flavipes, Reticulitermes lucifugus, Termes natalensis.

Aus der Ordnung der Geradflügler (Orthoptera) beispielsweise Acheta domestica, Blatta orientalis, Blattella germanica, Forficula auricularia, Gryllotalpa gryllotalpa, Locusta migratoria, Melanoplus bivittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus, Tachycines asynamorus.

Aus der Klasse der Arachnoidea beispielsweise Spinnentiere (Acarina) wie Amblyomma americanum, Amblyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Ornithodorus moubata, Otobius megnini, Paratetranychus pilosus, Dermanyssus gallinae, Phyllocoptruta oleivora, Polyphagotarsonemus latus, Psoroptes ovis, Rhipicephalus appendiculatus, Rhipicephalus evertsi, Sarcoptes scabiei, Tetranychus cinnabarinus, Tetranychus kanzawai, Tetranychus pacificus, Tetranychus telarius, Tetranychus urticae.

Aus der Klasse der Nematoden beispielsweise Wurzelgallennematoden, z.B. Meloidogyne hapla, Meloidogyne incognita, Meloidogyne javanica, Zysten bildende Nematoden, z.B. Globodera rostochiensis, Heterodera avenae, Heterodera glycines, Heterodera schachtii, Heterodera trifolii, Stock- und Blattalchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Pratylenchus curvitatus, Pratylenchus goodeyi.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Die Wirkstoffkonzentrationen in den anwendungsfertigen Zubereitungen können in größeren Bereichen variiert werden.

Im allgemeinen liegen sie zwischen 0,0001 und 10 %, vorzugsweise zwischen 0,01 und 1 %.

Die Wirkstoffe können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Die Aufwandmenge an Wirkstoff zur Bekämpfung von Schädlingen beträgt unter Freilandbedingungen 0,1 bis 2,0, vorzugsweise 0,2 bis 1,0 kg/ha.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Die Formulierungen enthalten im allgemeinen zwischen 0,01 und 95 Gew.%, vorzugsweise zwischen 0,1 und 90 Gew.% des Wirkstoffs. Die Wirkstoffe werden dabei in einer Reinheit von 90 % bis 100 %, vorzugsweise 95 % bis 100 % (nach NMR-Spektrum) eingesetzt.

Beispiele für Formulierungen sind:
I. 5 Gew.-Teile einer erfindungsgemäßen Verbindung werden mit 95 Gew.-Teilen feinteiligem Kaolin innig vermischt. Man erhält auf diese Weise ein Stäubemittel, das 5 Gew.% des Wirkstoffs enthält.
II. 30 Gew.-Teile einer erfindungsgemäßen Verbindung werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit (Wirkstoffgehalt 23 Gew.%).
III. 10 Gew.-Teile einer erfindungsgemäßen Verbindung werden in einer Mischung gelöst, die aus 90 Gew.-Teilen Xylol, 6 Gew.-Teilen des Anlagerungsproduktes von 8 bis 10 Mol Ethylenoxid an 1 Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.%).
IV. 20 Gew.-Teile einer erfindungsgemäßen Verbindung werden in einer Mischung gelost, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht (Wirkstoffgehalt 16 Gew.%).
V. 80 Gew.-Teile einer erfindungsgemaßen Verbindung werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen (Wirkstoffgehalt 80 Gew.%).
VI. Man vermischt 90 Gew.-Teile einer erfindungsgemäßen Verbindung mit 10 Gew.-Teilen N-Methyl-a-pyrrolidon und erhält eine Lösung, die zur Anwendung in Form kleinster Tropfen geeignet ist (Wirkstoffgehalt 90 Gew.%).
VII. 20 Gew.-Teile einer erfindungsgemäßen Verbindung werden in einer Mischung gelöst, die aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhalt man eine wäßrige Dispersion, die 0,02 Gew.% des Wirkstoffs enthält.
VIII.20 Gew.-Teile einer erfindungsgemäßen Verbindung werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20 000 Gew.-Teilen Wasser erhält man eine Spritzbrühe, die 0,1 Gew.% des Wirkstoffs enthält.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

### Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in der anschließenden Tabelle mit physikalischen Angaben aufgeführt.
1. N-Methoxy-N-(2-(1'-methyl-1'-methoximino-1''-phenyl-iminoxymethylphenyl)-carbaminsäuremethylester 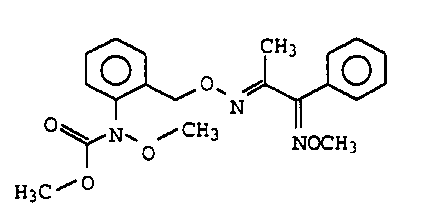
   a) N-Methoxy-N- (2-(1'-methyl-1'-benzoyl)-iminowmethylphenyl)-carbaminsäuremethylester 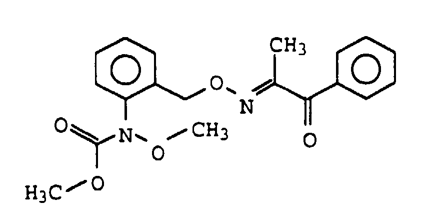 Eine Mischung von 25 g (0,15 ml) α-Hydroximino-propiophenonoxim, 42 g (0,12 ml) N-(2-Brommethylphenyl)-N-methoxy-carbaminsäuremethylester (WO 93/15046; Reinheit ca. 80 %ig) und 42,3 g (0,3 mol) K₂CO₃ in 100 ml Dimethylformamid wird über Nacht bei Raumtemperatur gerührt. Anschließend verdünnt man die Reaktionsmischung mit Wasser und extrahiert die waßrige Phase dreimal mit Methyl-t-butylether. Die vereinigten organischen Phasen werden einmal mit Wasser extrahiert, über MgSO₄ getrocknet und eingeengt. Der Rückstand wird säulenchromatographisch mit Cyclohexan/Essigester-Gemischen gereinigt. Man erhält 30 g (70 %) der Titelverbindung als hellgelbes Öl.
      ¹H-NMR (CDCl₃; δ in ppm):
      7,75 (d,1H,Phenyl); 7,4 (m,8H,Phenyl); 5,3 (s,2H,OCH₂),
      3,7 (s,3H,OCH₃); 3,6 (s,3H,OCH₃); 2,2 (s,3H,CH₃)
   b) N-Methoxy-N- (2-(1'-methyl-1'-methoximino-1''-phenyl)-iminoxymethylphenyl)-carbaminsäuremethylester 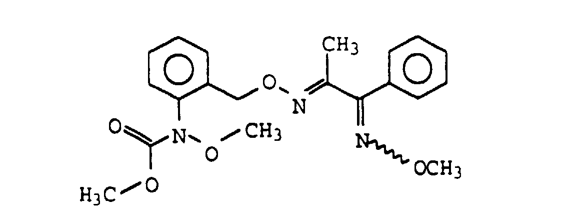 Eine Mischung von 0,8 g (10 ml) Methoxyamin-Hydrochlorid, 0,9 g (12 mol) Pyridin und 3 g des Ketons aus Beispiel la in 15 ml Methanol wird 3 Tage bei Raumtemperatur gerührt. Anschließend verdünnt man die Reaktionsmischung mit Wasser und extrahiert die wäßrige Phase dreimal mit Methylenchlorid. Die vereinigten organischen Phasen werden mit verdünnter Salzsäure-Lösung und Wasser extrahiert, über MgSO₄ getrocknet und eingeengt. Als Rückstand erhält man 1,5 g (47 %) der Titelverbindung (2 Isomere, ca. 2:1) als gelbes Öl.
      ¹H-NMR (CDCl₃; δ in ppm):
      Isomeres A (größere Menge): 5,25 (s,2H,OCH₂);
      4,0 (s,3H,OCH₃); 3,70 (s,3H,OCH₃); 3,6 (s,3H,OCH₃);
      2,15 (s,3H,CH₃)
      Isomeres B (kleinere Menge): 5,05 (s,2H,OCH₂;
      3,9 (s,3H,OCH₃); 3,68 (s,3H,OCH₃); 3,45 (s,3H,OCH₃);
      2,2 (s,3H,CH₃)
      Die Signale der aromatischen Protone der beiden Isomeren sind nicht aufgelöst:
      7,5 (d,breit); 7,2-7,4 (m); 7,15 (m)
2. N-Methyl-N'-methoxy-N'-(2-(1'-methyl-1'-methoximino-1''-ethyl)-iminoxymethylphenyl)-harnstoff 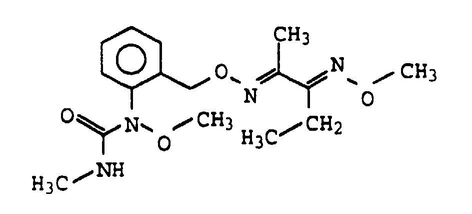
   a) N-Methyl-N'-methoxy-N'-(2-(1'-methyl-1'-propionyl)-iminoxymethylphenyl)-harnstoff 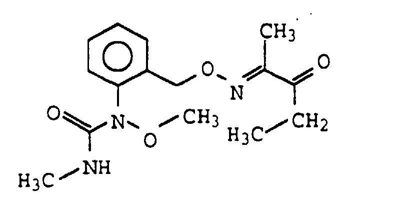
      Eine Mischung von 1,9 g (16 mmol) α-Hydroximinopentanon-3, 5 g (15 mmol) N-Methoxy-N-(2-brommethylphenyl)-carbaminsäurephenylester (hergestellt analog WO 93/15046) und 4,1 g (30 mmol) K₂CO₃ in 20 ml Dimethylformamid wird über Nacht bei Raumtemperatur gerührt. Anschließend verdünnt man die Reaktionsmischung mit Wasser und extrahiert die wäßrige Phase dreimal mit Methyl-t-butylether. Die vereinigten organischen Phasen werden über MgSO₄ getrocknet, über Al₂O₃ abgesaugt und eingeengt.
      Der Rückstand wird mit 20 ml 40 %iger wäßriger Methylamin-Lösung versetzt und über Nacht bei Raumtemperatur gerührt.
      Anschließend wird die Reaktionsmischung dreimal mit Methyl-t-butylether extrahiert. Die vereinigten organischen Phasen werden einmal mit Wasser extrahiert, über MgSO₄ getrocknet und eingeengt. Der Rückstand wird säulenchromatographisch mit Cyclohexan/Essigester-Gemischen gereinigt. Man erhält 2,7 g (59 %) der Titelverbindung als hellgelbes Öl.
      ¹H-NMR (CDCl₃; δ in ppm):
      7,45 (m,1H,Phenyl); 7,35 (m,3H,Phenyl);
      6,1 (s,breit,1H,NH); 5,4 (s,2H,OCH₂); 3,7 (s,3H,OCH₃);
      2,9 (d,3H,N-CH₃); 2,8 (q,2H,CH₂-CH₃); 2,0 (s,3H,CH₃); 1,1 (t,3H,CH₂-CH₃)
   b) N-Methyl-N'-methoxy-N'-(2-(1'-methyl-1'-methoximino-1''-ethyl)-iminoxymethylphenyl)-harnstoff 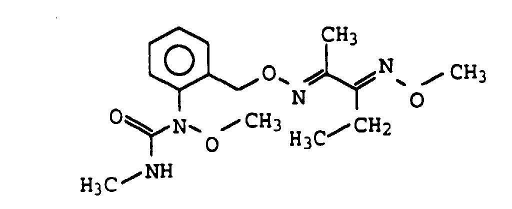
      Eine Mischung von 2,7 g (8,8 mmol) des Ketons aus Beispiel 2a und 1,5 g (18 mmol) Methoxyyamin·HCl in 15 ml Methanol wird über Nacht bei Raumtemperatur gerührt. Anschließend verdünnt man die Reaktionsmischung mit Wasser und extrahiert die wäßrige Phase dreimal mit Methyl-t-butylether. Die vereinigten organischen Phasen werden einmal mit Wasser extrahiert, über MgSO4 getrocknet und eingeengt. Der Rückstand kristallisiert und wird wird mit Hexan ausgerührt. Man erhält 1,5 g (51 %) der Titelverbindung als farblose Kristalle (Fp. = 92°C).
      ¹H-NMR (CDCl₃; δ in ppm):
      7,45 (m,1H,Phenyl); 7,3 (m,3H,Phenyl);
      6,0 (s,breit,1H,NH); 5,3 (s,2H,O-CH₂); 3,95 (s,3H,OCH₃);
      3,7 (s.3H,OCH₃); 2,9 (d,3H,N-CH₃); 2,5 (q,2H,CH₂-CH₃);
      2,05 (s,3H,CH₃); 0,95 (t,3H,CH₂-CH₃)

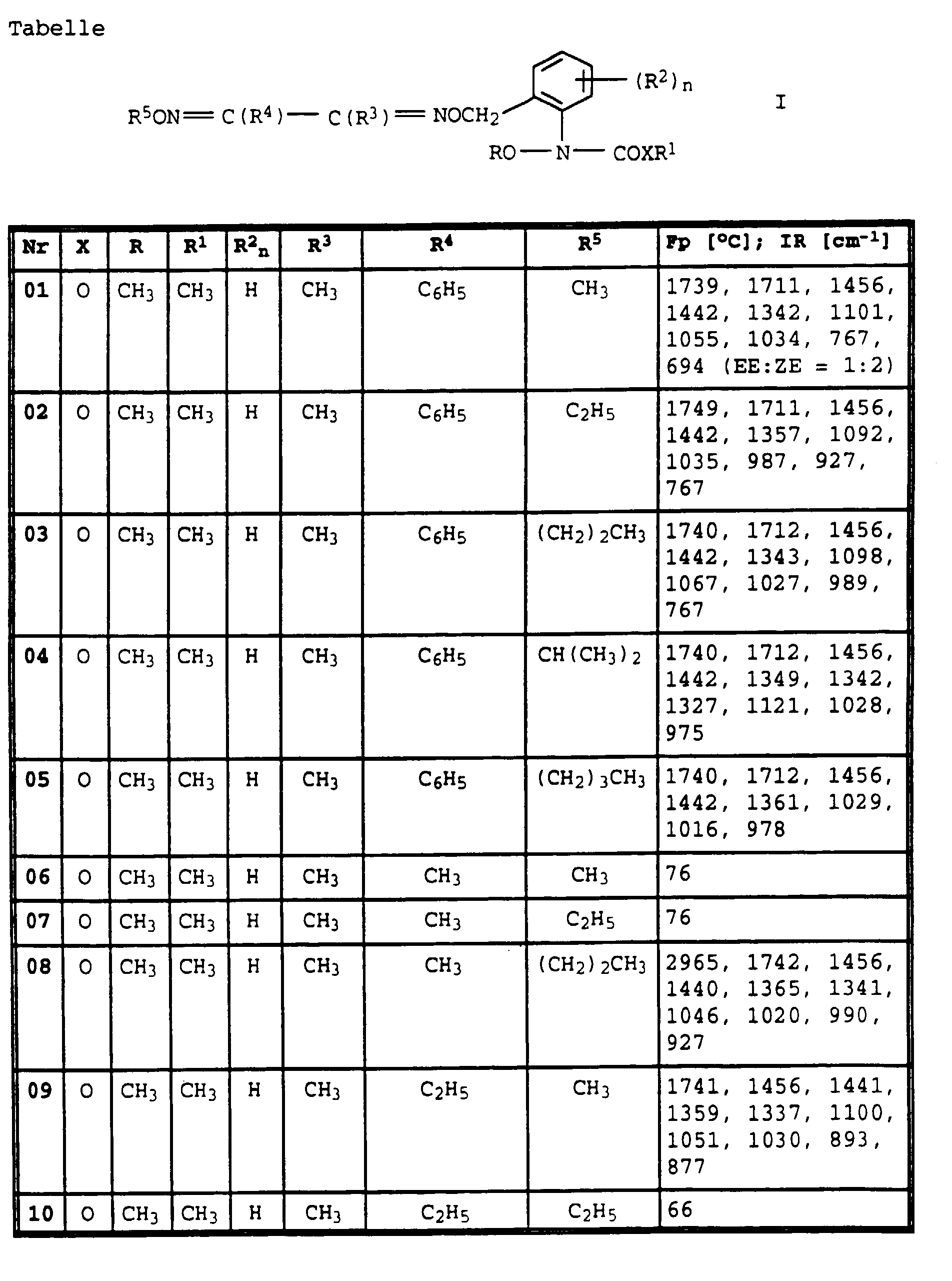

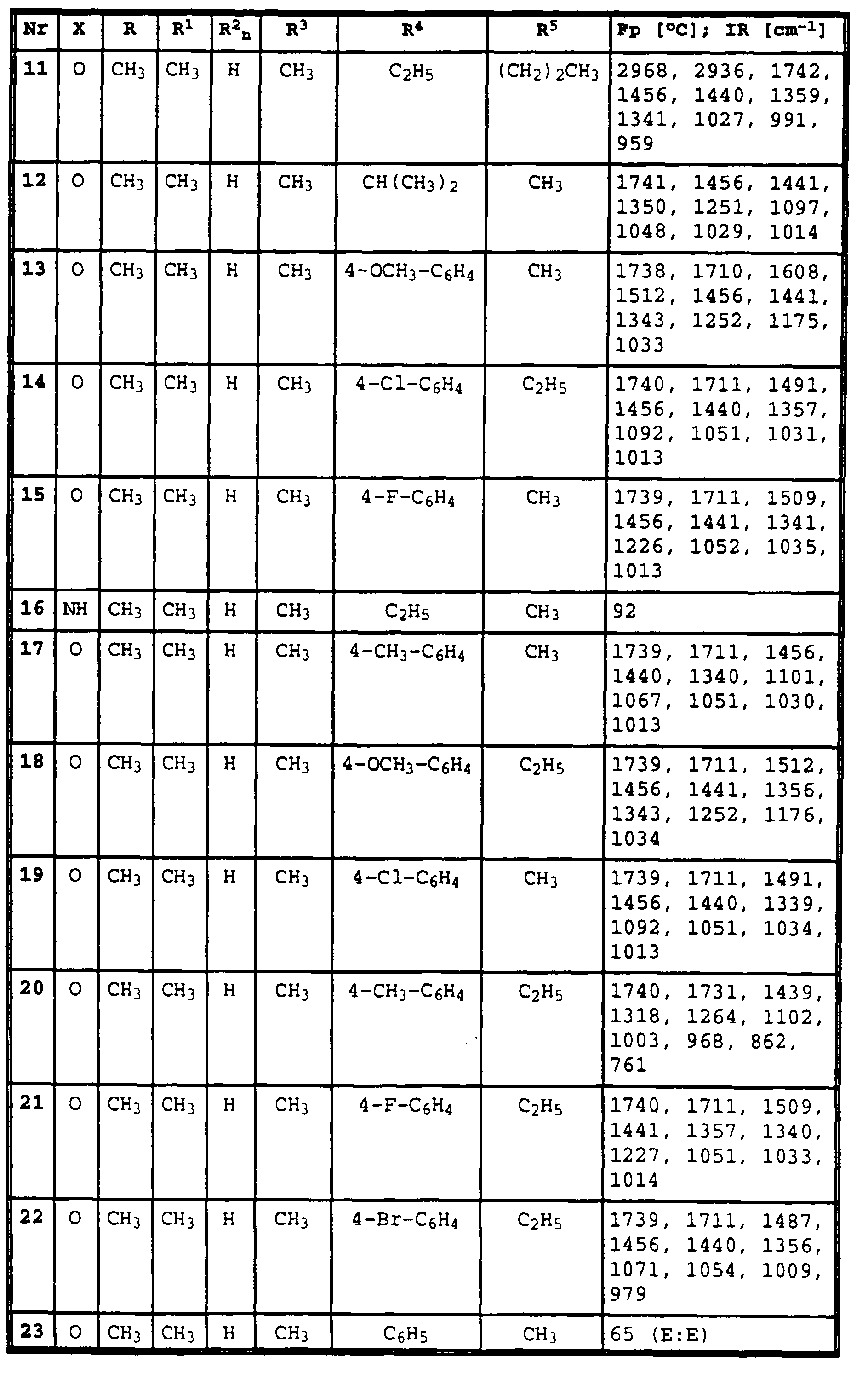

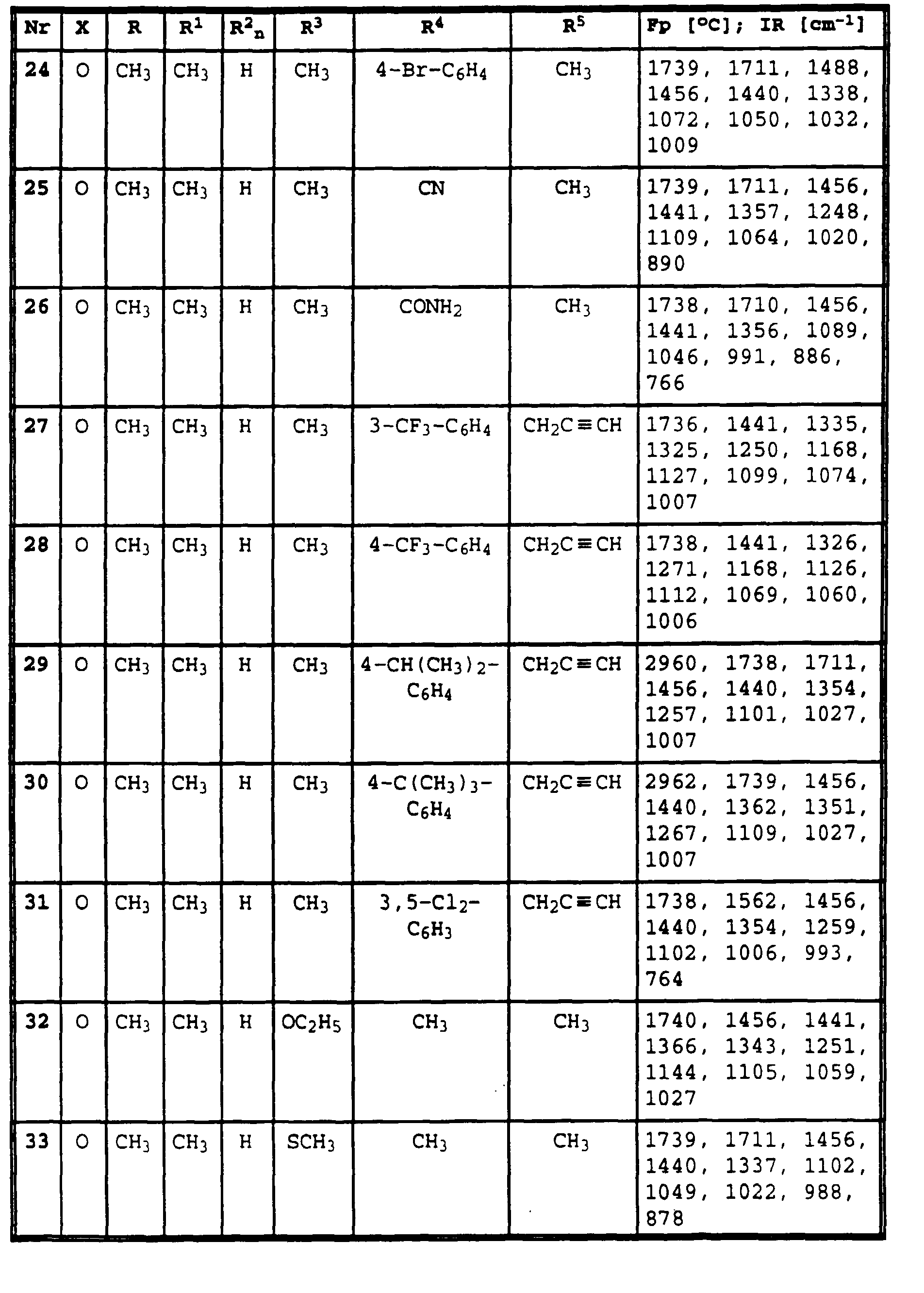

### Beispiele zur Wirkung gegen Schadpilze

Die fungizide Wirkung der Verbindungen der allgemeinen Formel I ließ sich durch folgende Versuche zeigen:

Die Wirkstoffe wurden als 20 %-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole) aufbereitet und entsprechend der gewünschten Konzentration mit Wasser verdünnt.

### Wirkung gegen Pyricularia oryzae (Reisbrand)

Reiskeimlinge (Sorte: "Tai Nong 67") wurden mit der Wirkstoffaufbereitung tropfnaß gespritzt. Nach 24 Stunden wurden die Pflanzen mit einer wäßrigen Sporensuspension des Pilzes *Pyricularia oryzae* besprüht und 6 Tage bei 22 bis 24°C bei einer relativen Luftfeuchtigkeit von 95 bis 99 % bewahrt. Die Beurteilung erfolgte visuell.

In diesem Test zeigten die mit 63 ppm der erfindungsgemäßen Verbindungen 01 bis 07 behandelten Pflanzen einen Befall von 15 % und weniger während die unbehandelten Pflanzen zu 60 % befallen waren.

In einem entsprechenden Test zeigten die mit 63 ppm der erfindungsgemäßen Verbindungen 08 bis 15, 17, 19 und 21 bis 24 behandelten Pflanzen einen Befall von 15 % und weniger, während die unbehandelten Pflanzen zu 60 % befallen waren.

### Wirkung gegen Puccinia recondita (Weizenbraunrost)

Blätter von Weizensämlingen (Sorte "Kanzler") wurden mit Sporen des Braunrosts *(Puccinia recondita)* bestäubt. Die so behandelten Pflanzen wurden 24 h bei 20 bis 22°C und einer relativen Luftfeuchtigkeit von 90 bis 95 % inkubiert und anschließend mit der wäßrigen Wirkstoffaufbereitung behandelt. Nach weiteren 8 Tagen bei 20 bis 22°C und 65-70% relativer Luftfeuchtigkeit wurde das Ausmaß der Pilzentwicklung ermittlelt. Die Auswertung erfolgte visuell.

In diesem Test zeigten die mit 250 ppm der erfindungsgemäßen Verbindungen 01 bis 05 und 07 behandelten Pflanzen einen Befall von 15 % und weniger, während die unbehandelten Pflanzen zu 70 % befallen waren.

In einem entsprechenden Test zeigten die mit 250 ppm der erfindungsgemäßen Verbindungen 08 bis 15 und 17 bis 24 behandelten Pflanzen einen Befall von 5 % und weniger, während die unbehandelten Pflanzen zu 70% befallen waren.

### Beispiele zur Wirkung gegen tierische Schädlinge

Die insektizide Wirkung der Verbindungen der allgemeinen Formel I ließ sich durch folgende Versuche zeigen:

Die Wirkstoffe wurden
a) als 0,1 %-ige Lösung in Aceton oder
b) als 10 %-ige Emulsion in einem Gemisch aus 70 Gew.-% Cyclohexanol, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL, Emulgator auf der Basis ethoxylierter Fettalkohole)
aufbereitet und entsprechend der gewünschten Konzentration mit Aceton im Fall von a) bzw. mit Wasser im Fall von b) verdünnt.

Nach Abschluß der Versuche wurde die jeweils niedrigste Konzentration ermittelt, bei der die Verbindungen im Vergleich zu unbehandelten Kontrollversuchen noch eine 80 - 100 %-ige Hemmung bzw. Mortalität hervorriefen (Wirkschwelle bzw. Minimalkonzentration).

## Patentansprüche

1. Iminooximethylenanilide der Formel I 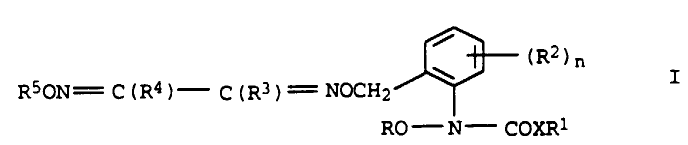 in der die Substituenten und der Index die folgende Bedeutung haben:
R Wasserstoff,
ggf. subst. Alkyl, Alkenyl, Alkinyl, Cycloalkyl, Cycloalkenyl, Cycloalkinyl, Alkylcarbonyl oder Alk-oxycarbonyl;
R¹ Alkyl, Alkenyl, Alkinyl, Cycloalkyl oder Cycloalkenyl und
für den Fall, daß X für NR^{a} steht, zusätzlich Wasserstoff;
X eine direkte Bindung, O oder NR^{a};
R^{a} Wasserstoff, Alkyl, Alkenyl, Alkinyl, Cycloalkyl oder Cycloalkenyl;
R² Cyano, Nitro, Trifluormethyl, Halogen, C₁-C₄-Alkyl oder C₁-C₄-Alkoxy;
n 0, 1 oder 2, wobei die Reste R² verschieden sein können, wenn n für 2 steht;
R³ Wasserstoff, Hydroxy, Cyano, Cyclopropyl, Trifluormethyl, Halogen, C₁-C₆-Alkyl, C₁-C₆-Alkoxy oder C₁-C₆-Alkylthio;
R⁴ Wasserstoff, Cyano, Nitro, Hydroxy, Amino, Halogen,
C₁-C₆-Alkyl, C₁-C₆-Alkoxy, C₁-C₆-Alkylthio, C₁-C₆-Alkylamino, Di-C₁-C₆-alkylamino, C₂-C₆-Alkenyl, C₂-C₆-Alkenyloxy, C₂-C₆-Alkenylthio, C₂-C₆-Alkenylamino, N-C₂-C₆-Alkenyl-N-C₁-C₆-alkylamino, C₂-C₆-Alkinyl, C₂-C₆-Alkinyloxy, C₂-C₆-Alkinylthio, C₂-C₆-Alkinylamino, N-C₂-C₆-Alkinyl-N-C₁-C₆-alkylamino, wobei die Kohlenwasserstoffreste dieser Gruppen partiell oder vollständig halogeniert sein können oder einen bis drei der folgenden Reste tragen können: Cyano, Nitro, Hydroxy, Merkapto,Amino, Carboxyl, Aminocarbonyl, Aminothiocarbonyl, Halogen, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₁-C₆-Alkylsulfonyl, C₁-C₆-Alkylsulfoxyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₂-C₆-Alkenyloxy, C₃-C₆-Cycloalkyl, C₃-C₆-Cycloalkyloxy, Heterocyclyl, Heterocyclyloxy, Aryl, Aryloxy, Aryl-C₁-C₄-alkoxy, Arylthio, Aryl-C₁-C₄-alkylthio, Hetaryl, Hetaryloxy, Hetaryl-C₁-C₄-alkoxy, Hetarylthio, Hetaryl-C₁-C₄-alkylthio, wobei die cyclischen Reste ihrerseits partiell oder vollständig halogeniert sein können und/oder ein bis drei der folgenden Gruppen tragen können: Cyano, Nitro, Hydroxy, Mercapto, Amino, Carboxyl, Aminocarbonyl, Aminothiocarbonyl, C₁-C₆-Alkyl, C₁-C₆-Halogenalkyl, C₁-C₆-Alkylsulfonyl, C₁-C₆-Alkylsulfoxyl, C₃C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyloxy, Aryl, Aryloxy, Arylthio, Hetaryl, Hetaryloxy, Hetarylthio und C (=NOR⁶)-Aₙ-R⁷;
C₃-C₆-Cycloalkyl, C₃-C₆-Cycloalkyloxy, C₃-C₆-Cycloalkylthio, C₃-C₆-Cycloalkylamino, N-C₃-C₆-Cycloalkyl-N-C₁-C₆-alkylamino, C₃-C₆-Cycloalkenyl, C₃-C₆-cycloalkenyloxy, C₃-C₆-Cycloalkenylthio, C₃-C₆-Cycloalkenylamino, N-C₃-C₆-Cycloalkenyl-N-C₁-C₆-alkylamino, Heterocyclyl, Heterocyclyloxy, Heterocyclylthio, Heterocyclylamino, N-Heterocyclyl-N-C₁-C₆-alkylamino, Aryl, Aryloxy, Arylthio, Arylamino, N-Aryl-N-C₁-C₆-alkylamino, Hetaryl, Hetaryloxy, Hetarylthio, Hetarylamino, N-Hetaryl-N-C₁-C₆-alkylamino, wobei die cyclischen Reste partiell oder vollständig halogeniert sein können oder einen bis drei der folgenden Gruppen tragen können: Cyano, Nitro, Hydroxy, Mercapto, Amino, Carboxyl, Aminocarbonyl, Aminothiocarbonyl, Halogen, C₁-C₆-Alkyl, C₁-C₆-Halogenalkyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyloxy, Aryl, Aryloxy, Hetaryl und Hetaryloxy;
R⁵ Wasserstoff,
C₁-C₁₀-Alkyl, C₃-C₆-Cycloalkyl, C₂-C₁₀-Alkenyl, C₂-C₁₀-Alkinyl, C₁-C₁₀-Alkylcarbonyl, C₂-C₁₀-Alkenylcarbonyl, C₃-C₁₀-Alkinylcarbonyl oder C₁-C₁₀-Alkylsulfonyl, wobei diese Reste partiell oder vollständig halogeniert sein können oder einen bis drei der folgenden Gruppen tragen können: Cyano, Nitro, Hydroxy, Mercapto, Amino, Carboxyl, Aminocarbonyl, Aminothiocarbonyl, Halogen, C₁-C₆-Alkyl, C₁-C₆-Halogenalkyl, C₁-C₆-Alkylsulfonyl, C₁-C₆-Alkylsulfoxyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, C₃-C₆-Cycloalkyl, C₃-C₆-Cycloalkyloxy, Heterocyclyl, Heterocyclyloxy, Benzyl, Benzyloxy, Aryl, Aryloxy, Arylthio, Hetaryl, Hetaryloxy und Hetarylthio, wobei die cyclischen Gruppen ihrerseits partiell oder vollständig halogeniert sein können oder einen bis drei der folgenden Gruppen tragen können: Cyano, Nitro, Hydroxy, Mercapto, Amino, Carboxyl, Aminocarbonyl, Aminothiocarbonyl, Halogen, C₁-C₆-Alkyl, C₁-C₆-Halogenalkyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkyloxycarbonyl, C₁-C₆-Alkylthio, C₁-C₆-Alkylamino, Di-C₁-C₆-Alkylamino, C₁-C₆-Alkylaminocarbonyl, Di-C₁-C₆-Alkylaminocarbonyl, C₁-C₆-Alkylaminothiocarbonyl, Di-C₁-C₆-Alkylaminothiocarbonyl, C₂-C₆-Alkenyl, C₂-C₆-Alkenyloxy, Benzyl, Benzyloxy, Aryl, Aryloxy, Arylthio, Hetaryl, Hetaryloxy, Hetarylthio oder C (=NOR⁶) -Aₙ-R⁷;
Aryl, Arylcarbonyl, Arylsulfonyl, Hetaryl, Hetarylcarbonyl oder Hetarylsulfonyl, wobei diese Reste partiell oder vollständig halogeniert sein können oder einen bis drei der folgenden Gruppen tragen können: Cyano, Nitro, Hydroxy, Mercapto, Amino, Carboxyl, Aminocarbonyl, Aminothiocarbonyl, Halogen, C₁-C₆-Alkyl, C₁-C₆-Halogenalkyl, C₁-C₆-Alkylcarbonyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkyloxycarbonyl, C₁-C₆-Alkylthio, C₁-C₆-Alkylamino, Di-C₁-C₆-Alkylamino, C₁-C₆-Alkylaminocarbonyl, Di-C₁-C₆-Alkylaminocarbonyl, C₁-C₆-Alkylaminothiocarbonyl, Di-C₁-C₆-Alkylaminothiocarbonyl, C₂-C₆-Alkenyl, C₂-C₆-Alkenyloxy, Benzyl, Benzyloxy, Aryl, Aryloxy, Hetaryl, Hetaryloxy oder C(=NOR⁶)-Aₙ-R⁷;
wobei
A für Sauerstoff, Schwefel oder Stickstoff steht und wobei der Stickstoff Wasserstoff oder C₁-C₆-Alkyl trägt;
m 0 oder 1;
R⁶ Wasserstoff, C₁-C₆-Alkyl, C₂-C₆-Alkenyl oder C₂-C₆-Alkinyl bedeutet und
R⁷ Wasserstoff, C₁-C₆-Alkyl, C₂-C₆-Alkenyl oder C₂-C₆-Alkinyl bedeutet,
sowie deren Salze.

2. Verbindungen der Formel I gemäß Anspruch 1, in denen n für 0 steht.

3. Verbindungen der Formel I gemäß Anspruch 1, in denen R¹ für Methyl steht.

4. Verfahren zur Herstellung der Verbindungen der Formel I gemäß Anspruch 1, in denen R nicht Wasserstoff und R³ nicht Halogen bedeutet, dadurch gekennzeichnet, daß man ein Benzylderivat der Formel II 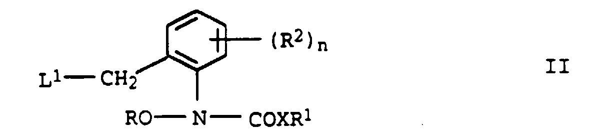 in der L¹ für eine nucleophil austauschbare Abgangsgruppe steht, in an sich bekannter Weise mit einem Oxim der Formel III
R⁵ON=C(R⁴)-C(R³) = NOH III
umsetzt.

5. Verfahren zur Herstellung der Verbindungen der Formel I gemäß Anspruch 1, in denen R nicht Wasserstoff und R³ und R⁴ nicht Halogen bedeutet, dadurch gekennzeichnet, daß man ein Benzylderivat der Formel II gemäß Anspruch 1 in an sich bekannter Weise mit einem Dioxim der Formel IV
HON= C(R⁴) - C(R³) = NOH IV
zu einer Verbindung der Formel V 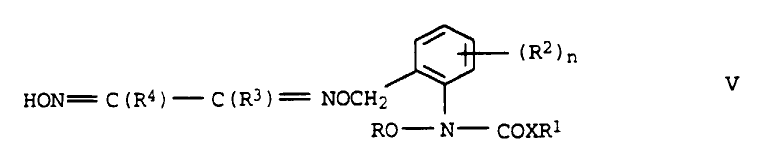 umsetzt und V anschließend mit einer Verbindung der Formel VI
R⁵ - L² VI
in der L² für eine nucleophil austauschbare Abgangsgruppe steht, zu I umsetzt.

6. Verfahren zur Herstellung der Verbindungen der Formel I gemäß Anspruch 1, in denen R³ nicht Halogen bedeutet, dadurch gekennzeichnet, daß man ein Benzylderivat der Formel II gemäß Anspruch 1 in an sich bekannter Weise mit einem Ketooxim der Formel VII
O= C(R⁴) - C(R³) = NOH VII
zu einer Verbindung der Formel VIII 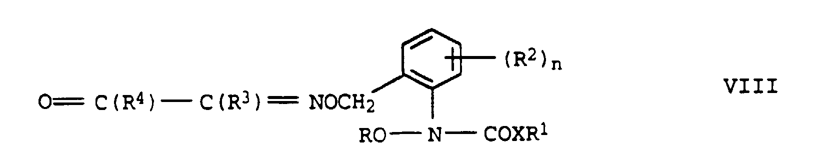 umsetzt, VIII anschließend entweder
a) zunächst mit Hydroxylamin oder dessen Salz und danach mit einer Verbindung der Formel VI (R⁵-L²) gemäß Anspruch 5 oder
b) mit einem Hydroxylamin oder einem Hydroxylammoniumsalz der Formel IXa bzw. IXb
R⁵ - ONH₂ IXa
R⁵ - ONH₃^{⊕} Q^{⊖} IXb
in der Q^{⊖} für das Anion einer Säure steht,
zu I umsetzt.

7. Mittel gegen tierische Schädlinge oder Schadpilze, enthaltend übliche Zusatzstoffe und eine wirksame Menge einer Verbindung der Formel I gemäß Anspruch 1.

8. Mittel nach Anspruch 7 zur Bekämpfung tierischer Schädlinge aus der Klasse der Insekten, Spinntiere oder Nematoden.

9. Verfahren zur Bekämpfung von tierischen Schädlingen oder Schadpilzen, dadurch gekennzeichnet, daß man die Schädlinge oder Schadpilze, deren Lebensraum oder die von ihnen freizuhaltenden Pflanzen, Flächen, Materialien oder Räume mit einer wirksamen Menge einer Verbindung der Formel I gemäß Anspruch 1 behandelt.

10. Verwendung der Verbindungen I gemäß Anspruch 1 zur Herstellung von Mitteln gegen tierische Schädlinge oder Schadpilze.

11. Verwendung der Verbindungen I gemäß Anspruch 1 zur Bekämpfung von tierischen Schädlingen oder Schadpilzen.

12. Verbindungen der Formel VIII gemäß Anspruch 6.

13. Verwendung der Verbindungen der Formel VIII gemäß Anspruch 6 als Zwischenprodukte.

14. Verbindungen der Formel X 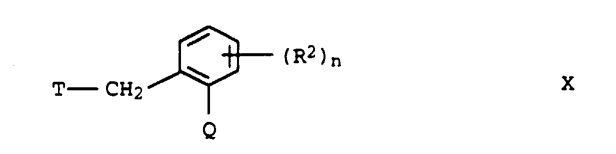 in der der Index und die Substituenten die folgende Bedeutung haben:
Q NO₂, NHOH und N(OR)-CO₂C₆H₅;
T O=C(R⁴)-C(R³)=NO-, HON=C(R⁴)-C(R³)=NO- oder R⁵ON=C (R⁴)-C(R³) =NO-,
wobei die Substituenten R, R¹, R², R³, R⁴ und R⁵ und der Index n die in Anspruch 1 gegebene Bedeutung haben.

15. Verwendung der Verbindungen der Formel X gemäß Anspruch 14 als Zwischenprodukte.

16. Verbindungen der Formel XI 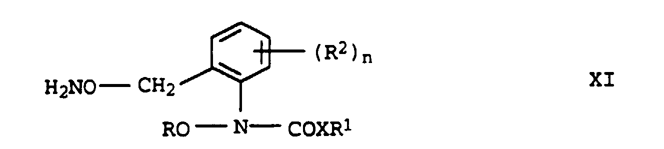 in der der Index n und die Substituenten R, R¹ und R² die in Anspruch 1 gegebene Bedeutung haben.

17. Verwendung der Verbindungen der Formel XI gemäß Anspruch 16 als Zwischenprodukte.

## Claims

1. An iminooxymethylenanilide of the formula I 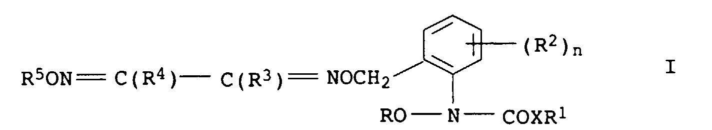 where the substituents and the index have the following meanings:
R is hydrogen,
unsubst. or subst. alkyl, alkenyl, alkynyl, cycloalkyl, cycloalkenyl, cycloalkynyl, alkylcarbonyl or alkoxycarbonyl;
R¹ is alkyl, alkenyl, alkynyl, cycloalkyl or cycloalkenyl and
in the case where X is NR^{a}, additionally hydrogen;
X is a direct bond, O or NR^{a};
R^{a} is hydrogen, alkyl, alkenyl, alkynyl, cycloalkyl or cycloalkenyl;
R² is cyano, nitro, trifluoromethyl, halogen, C₁-C₄-alkyl or C₁-C₄-alkoxy;
n is 0, 1 or 2, it being possible for the radicals R² to be different if n is 2;
R³ is hydrogen, hydroxyl, cyano, cyclopropyl, trifluoromethyl, halogen, C₁-C₆-alkyl, C₁-C₆-alkoxy or C₁-C₆-alkylthio;
R⁴ is hydrogen, cyano, nitro, hydroxyl, amino, halogen,
C₁-C₆-alkyl, C₁-C₆-alkoxy, C₁-C₆-alkylthio, C₁-C₆-alkylamino, di-C₁-C₆-alkylamino, C₂-C₆-alkenyl, C₂-C₆-alkenyloxy, C₂-C₆-alkenylthio, C₂-C₆-alkenylamino, N-C₂-C₆-alkenyl-N-C₁-C₆-alkylamino, C₂-C₆-alkynyl, C₂-C₆-alkynyloxy, C₂-C₆-alkynylthio, C₂-C₆-alkynylamino, N-C₂-C₆-alkynyl-N-C₁-C₆-alkylamino, it being possible for the hydrocarbon radicals of these groups to be partially or completely halogenated or to carry one to three of the following radicals: cyano, nitro, hydroxyl, mercapto, amino, carboxyl, aminocarbonyl, aminothiocarbonyl, halogen, C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₁-C₆-alkylsulfonyl, C₁-C₆-alkylsulfoxyl, C₁-C₆-alkoxy, C₁-C₆-haloalkoxy, C₁-C₆-alkoxycarbonyl, C₁-C₆-alkylthio, C₁-C₆-alkylamino, di-C₁-C₆-alkylamino, C₂-C₆-alkenyloxy, C₃-C₆-cycloalkyl, C₃-C₆-cycloalkyloxy, heterocyclyl, heterocyclyloxy, aryl, aryloxy, aryl-C₁-C₄-alkoxy, arylthio, aryl-C₁-C₄-alkylthio, hetaryl, hetaryloxy, hetaryl-C₁-C₄-alkoxy, hetarylthio, hetaryl-C₁-C₄-alkylthio, it being possible for the cyclic radicals in turn to be partially or completely halogenated and/or to carry one to three of the following groups: cyano, nitro, hydroxyl, mercapto, amino, carboxyl, aminocarbonyl, aminothiocarbonyl, C₁-C₆-alkyl, C₁-C₆-haloalkyl, C₁-C₆-alkylsulfonyl, C₁-C₆-alkylsulfoxyl, C₃-C₆-cycloalkyl, C₁-C₆-alkoxy, C₁-C₆-haloalkoxy, C₁-C₆-alkoxycarbonyl, C₁-C₆-alkylthio, C₁-C₆-alkylamino, di-C₁-C₆-alkylamino, C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₂-C₆-alkenyl, C₂-C₆-alkenyloxy, benzyl, benzyloxy, aryl, aryloxy, arylthio, hetaryl, hetaryloxy, hetarylthio and C (=NOR⁶)-Aₙ-R⁷;
C₃-C₆-cycloalkyl, C₃-C₆-cycloalkyloxy, C₃-C₆-cycloalkylthio, C₃-C₆-cycloalkylamino, N-C₃-C₆-cycloalkyl-N-C₁-C₆-alkylamino, C₃-C₆-cycloalkenyl, C₃-C₆-cycloalkenyloxy, C₃-C₆-cycloalkenylthio, C₃-C₆-cycloalkenylamino, N-C₃-C₆-cycloalkenyl-N-C₁-C₆-alkylamino, heterocyclyl, heterocyclyloxy, heterocyclylthio, heterocyclylamino, N-heterocyclyl-N-C₁-C₆-alkylamino, aryl, aryloxy, arylthio, arylamino, N-aryl-N-C₁-C₆-alkylamino, hetaryl, hetaryloxy, hetarylthio, hetarylamino, N-hetaryl-N-C₁-C₆-alkylamino, it being possible for the cyclic radicals to be partially or completely halogenated or to carry one to three of the following groups: cyano, nitro, hydroxyl, mercapto, amino, carboxyl, aminocarbonyl, aminothiocarbonyl, halogen, C₁-C₆-alkyl, C₁-C₆-haloalkyl, C₁-C₆-alkylsulfonyl, C₁-C₆-alkylsulfoxyl, C₃-C₆-cycloalkyl, C₁-C₆-alkoxy, C₁-C₆-haloalkoxy, C₁-C₆-alkoxycarbonyl, C₁-C₆-alkylthio, C₁-C₆-alkylamino, di-C₁-C₆-alkylamino, C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₂-C₆-alkenyl, C₂-C₆-alkenyloxy, benzyl, benzyloxy, aryl, aryloxy, hetaryl and hetaryloxy;
R⁵ is hydrogen,
C₁-C₁₀-alkyl, C₃-C₆-cycloalkyl, C₂-C₁₀-alkenyl, C₂-C₁₀-alkynyl, C₁-C₁₀-alkylcarbonyl, C₂-C₁₀-alkenylcarbonyl, C₃-C₁₀-alkynylcarbonyl or C₁-C₁₀-alkylsulfonyl, it being possible for these radicals to be partially or completely halogenated or to carry one to three of the following groups: cyano, nitro, hydroxyl, mercapto, amino, carboxyl, aminocarbonyl, aminothiocarbonyl, halogen, C₁-C₆-alkyl, C₁-C₆-haloalkyl, C₁-C₆-alkylsulfonyl, C₁-C₆-alkylsulfoxyl, C₁-C₆-alkoxy, C₁-C₆-haloalkoxy, C₁-C₆-alkoxycarbonyl, C₁-C₆-alkylthio, C₁-C₆-alkylamino, di-C₁-C₆-alkylamino, C₁-C₆-alkylaminocarbonyl, di- C₁-C₆-alkylaminocarbonyl, C₁-C₆-alkylaminothiocarbonyl, di-C₁-C₆-alkylaminothiocarbonyl, C₂-C₆-alkenyl, C₂-C₆-alkenyloxy, C₃-C₆-cycloalkyl, C₃-C₆-cycloalkyloxy, heterocyclyl, heterocyclyloxy, benzyl, benzyloxy, aryl, aryloxy, arylthio, hetaryl, hetaryloxy and hetarylthio, it being possible for the cyclic groups in turn to be partially or completely halogenated or to carry one to three of the following groups: cyano, nitro, hydroxyl, mercapto, amino, carboxyl, aminocarbonyl, aminothiocarbonyl, halogen, C₁-C₆-alkyl, C₁-C₆-haloalkyl, C₁-C₆-alkylsulfonyl, C₁-C₆-alkylsulfoxyl, C₃-C₆-cycloalkyl, C₁-C₆-alkoxy, C₁-C₆-haloalkoxy, C₁-C₆-alkyloxycarbonyl, C₁-C₆-alkylthio, C₁-C₆-alkylamino, di-C₁-C₆-alkylamino, C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₂-C₆-alkenyl, C₂-C₆-alkenyloxy, benzyl, benzyloxy, aryl, aryloxy, arylthio, hetaryl, hetaryloxy, hetarylthio or C(=NOR⁶)-Aₙ-R⁷;
aryl, arylcarbonyl, arylsulfonyl, hetaryl, hetarylcarbonyl or hetarylsulfonyl, it being possible for these radicals to be partially or completely halogenated or to carry one to three of the following groups: cyano, nitro, hydroxyl, mercapto, amino, carboxyl, aminocarbonyl, aminothiocarbonyl, halogen, C₁-C₆-alkyl, C₁-C₆-haloalkyl, C₁-C₆-alkylcarbonyl, C₁-C₆-alkylsulfonyl, C₁-C₆-alkylsulfoxyl, C₃-C₆-cycloalkyl, C₁-C₆-alkoxy, C₁-C₆-haloalkoxy, C₁-C₆-alkyloxycarbonyl, C₁-C₆-alkylthio, C₁-C₆-alkylamino, di-C₁-C₆-alkylamino, C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₂-C₆-alkenyl, C₂-C₆-alkenyloxy, benzyl, benzyloxy, aryl, aryloxy, hetaryl, hetaryloxy or C(=NOR⁶)-Aₙ-R⁷;
A being oxygen, sulfur or nitrogen and the nitrogen carrying hydrogen or C₁-C₆-alkyl;
m being 0 or 1;
R⁶ being hydrogen, C₁-C₆-alkyl, C₂-C₆-alkenyl or C₂-C₆-alkynyl and
R⁷ being hydrogen, C₁-C₆-alkyl, C₂-C₆-alkenyl or C₂-C₆-alkynyl,
or its salts.

2. A compound of the formula I as claimed in claim 1, where n is 0.

3. A compound of the formula I as claimed in claim 1, where R¹ is methyl.

4. A process for preparing the compounds of the formula I as claimed in claim 1, where R is not hydrogen and R³ is not halogen, which comprises reacting a benzyl derivative of the formula II 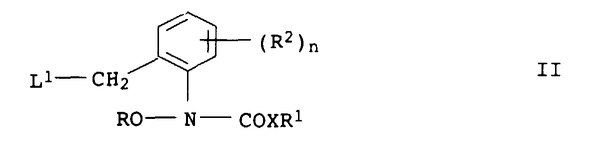 where L¹ is a nucleophilically replaceable leaving group, in a manner known per se with an oxime of the formula III
R⁵ON= C(R⁴) - C(R³) = NOH III.

5. A process for preparing the compounds of the formula I as claimed in claim 1, where R is not hydrogen and R³ and R⁴ are not halogen, which comprises reacting a benzyl derivative of the formula II as given in claim 4 in a manner known per se with a dioxime of the formula IV
HON= C(R⁴) - C(R³) = NOH IV
to give a compound of the formula V 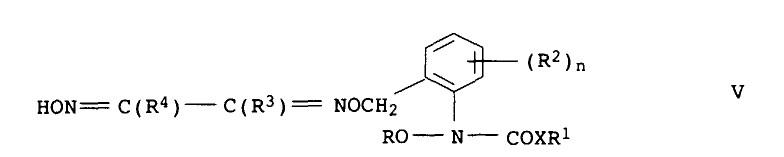 and then reacting V with a compound of the formula VI
R⁵ - L² VI
where L² is a nucleophilically replaceable leaving group, to give I.

6. A process for preparing the compounds of the formula I as claimed in claim 1, where R³ is not halogen, which comprises reacting a benzyl derivative of the formula II as given in claim 4 in a manner known per se with a ketoxime of the formula VII
O= C(R⁴) - C(R³)= NOH VII
to give a compound of the formula VIII 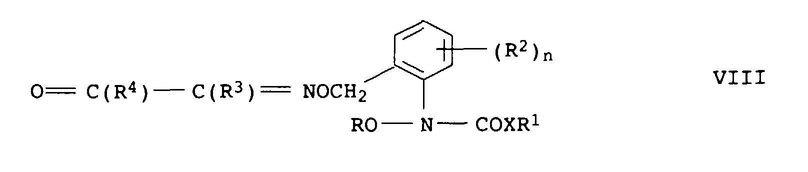 then reacting VIII either
a) first with hydroxylamine or its salt and then with a compound of the formula VI (R⁵-L²) as given in claim 5 or
b) with a hydroxylamine or a hydroxylammonium salt of the formula IXa or IXb
R⁵ - ONH₂ IXa
R⁵ - ONH₃^{⊕} Q^{⊖} IXb
where Q^{⊖} is the anion of an acid,
to give I.

7. A composition against animal pests or harmful fungi, containing customary additives and an effective amount of a compound of the formula I as claimed in claim 1.

8. A composition as claimed in claim 7 for controlling animal pests of the insects, arachnids or nematodes class.

9. A method of controlling animal pests or harmful fungi, which comprises treating the pests or harmful fungi, their habitat or the plants, surfaces, materials or spaces to be kept free from them with an effective amount of a compound of the formula I as claimed in claim 1.

10. The use of the compounds I as claimed in claim 1 for preparing compositions against animal pests or harmful fungi.

11. The use of the compounds I as claimed in claim 1 for controlling animal pests or harmful fungi.

12. A compound of the formula VIII as given in claim 6.

13. The use of the compounds of the formula VIII as given in claim 6 as intermediates.

14. A compound of the formula X 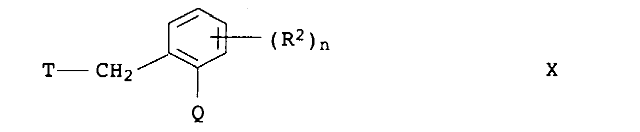 where the index and the substituents have the following meanings:
Q is NO₂, NHOH or N(OR)-CO₂C₆H₅;
T is O=C(R⁴)-C(R³)=NO-, HON=C(R⁴)-C(R³)=NO- or R⁵ON=C(R⁴)-C(R³)=NO-,
the substituents R, R¹, R², R³, R⁴ and R⁵ and the index n having the meanings given in claim 1.

15. The use of the compounds of the formula X as claimed in claim 14 as intermediates.

16. A compound of the formula XI 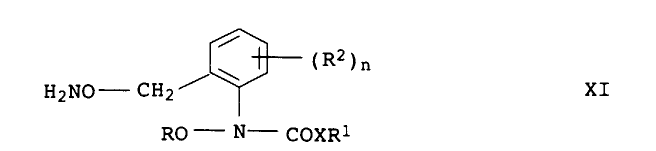 where the index n and the substituents R, R¹ and R² have the meanings given in claim 1.

17. The use of the compounds of the formula XI as claimed in claim 16 as intermediates.

## Revendications

1. Iminooxyméthylèneanilides de formule I 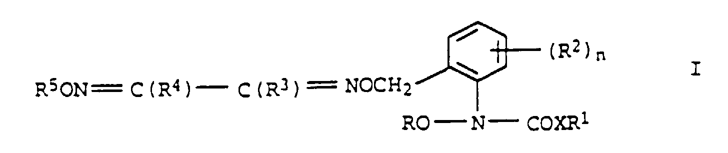 dans laquelle les symboles et l'indice ont les significations suivantes :
R : l'hydrogène,
un groupe alkyle éventuellement substitué, alcényle, alcynyle, cycloalkyle, cycloalcényle, cycloalcynyle, alkylcarbonyle ou alcoxycarbonyle ;
R¹ : un groupe alkyle, alcényle, alcynyle, cycloalkyle ou cycloalcényle et en outre, lorsque X représente NR^{a}, l'hydrogène ;
X : une liaison directe, O ou NR^{a} ;
R^{a} : l'hydrogène, un groupe alkyle, alcényle, alcynyle, cycloalkyle ou cycloalcényle ;
R² : un groupe cyano, nitro, trifluorométhyle, un halogène, un groupe alkyle en C1-C4 ou alcoxy en C1-C4 ;
n : 0, 1 ou 2, les substituants R² pouvant être différents lorsque n est égal à 2 ;
R³ : l'hydrogène, un groupe hydroxy, cyano, cyclopropyle, trifluorométhyle, un halogène, un groupe alkyle en C1-C6, alcoxy en C1-C6 ou alkylthio en C1-C6 ;
R⁴ : l'hydrogène, un groupe cyano, nitro, hydroxy, amino, un halogène, un groupe alkyle en C1-C6, alcoxy en C1-C6, alkylthio en C1-C6, alkylamino en C1-C6, di-(alkyle en C1-C6)amino, alcényle en C2-C6, alcényloxy en C2-C6, alcénylthio en C2-C6, alcénylamino en C2-C6, N-(alcényle en C2-C6)-N-(alkyle en C1-C6)amine, alcynyle en C2-C6, alcynyloxy en C2-C6, alcynylthio en C2-C6, alcynylamino en C2-C6, N-(alcynyle en C2-C6)-N-(alkyle en C1-C6)amine, les radicaux hydrocarbonés de ces groupes pouvant être halogénés en totalité ou en partie ou pouvant porter un à trois des substituants suivants : cyano, nitro, hydroxy, mercapto, amino, carboxyle, aminocarbonyle, aminothiocarbonyle, halogéno, (alkyle en C1-C6)aminocarbonyle, di(alkyle en C1-C6)aminocarbonyle, (alkyle en C1-C6)aminothiocarbonyle, di-(alkyle en C1-C6)aminothiocarbonyle, alkylsulfonyle en C1-C6, alkylsulfoxyde en C1-C6, alcoxy en C1-C6, halogénoalcoxy en C1-C6, (alcoxy en C1-C6)carbonyle, alkylthio en C1-C6, alkylamino en C1-C6, di-(alkyle en C1-C6)amino, alcényloxy en C2-C6, cycloalkyle en C3-C6, cycloalkyloxy en C3-C6, hétérocyclyle, hétérocyclyloxy, aryle, aryloxy, aryl-alcoxy en C1-C4, arylthio, aryl-alkylthio en C1-C4, hétéroaryle, hétéroaryloxy, hétéroaryl-alcoxy en C1-C4, hétéroarylthio, hétéroaryl-alkylthio en C1-C4, les groupes cycliques pouvant eux-mêmes êtres halogénés en totalité ou en partie et/ou pouvant porter un à trois des substituants suivants : cyano, nitro, hydroxy, mercapto, amino, carboxyle, aminocarbonyle, aminothiocarbonyle, alkyle en C1-C6, halogénoalkyle en C1-C6, alkylsulfonyle en C1-C6, alkylsulfoxyde en C1-C6, cycloalkyle en C3-C6, alcoxy en C1-C6, halogénoalcoxy en C1-C6, (alcoxy en C1-C6)carbonyle, alkylthio en C1-C6, alkylamino en C1-C6, di-(alkyle en C1-C6)amino, (alkyle en Cl-C6)aminocarbonyle, di-(alkyle en C1-C6)aminocarbonyle, (alkyle en C1-C6)aminothiocarbonyle, di-(alkyle en C1-C6)aminothiocarbonyle, alcényle en C2-C6, alcényloxy en C2-C6, benzyle, benzyloxy, aryle, aryloxy, arylthio, hétéroaryle, hétéroaryloxy, hétéroarylthio et C(=NOR⁶)-Aₘ-R⁷ ;
un groupe cycloalkyle en C3-C6, cycloalkyloxy en C3-C6, cycloalkylthio en C3-C6, cycloalkylamino en C3-C6, N-(cycloalkyle en C3-C6)-N-(alkyle en C1-C6)amino, cycloalcényle en C3-C6, cycloalcényloxy en C3-C6, cycloalcénylthio en C3-C6, cycloalcénylamino en C3-C6, N-(cycloalcényle en C3-C6)-N-(alkyle en C1-C6)amino, hétérocyclyle, hétérocyclyloxy, hétérocyclylthio, hétérocyclylamino, N-hétérocyclyl-N-(alkyle en C1-C6)amine, aryle, aryloxy, arylthio, arylamino, N-aryl-N-(alkyle en C1-C6)amine, hétéroaryle, hétéroaryloxy, hétéroarylthio, hétéroarylamino, N-hétéroaryl-N-(alkyle en C1-C6)amino, les radicaux cycliques pouvant être halogénés en totalité ou en partie ou pouvant porter un à trois des substituants suivants : cyano, nitro, hydroxy, mercapto, amino, carboxyle, aminocarbonyle, aminothiocarbonyle, halogéno, alkyle en C1-C6, halogénoalkyle en C1-C6, alkylsulfonyle en C1-C6, alkylsulfoxyde en C1-C6, cycloalkyle en C3-C6, alcoxy en C1-C6, halogénoalcoxy en C1-C6, (alcoxy en C1-C6)carbonyle, alkylthio en C1-C6, alkylamino en C1-C6, di-(alkyle en Cl-C6)amino, (alkyle en C1-C6)aminocarbonyle, di-(alkyle en C1-C6)aminocarbonyle, (alkyle en C1-C6)aminothiocarbonyle, di-(alkyle en C1-C6)aminothiocarbonyle, alcényle en C2-C6, alcényloxy en C2-C6, benzyle, benzyloxy, aryle, aryloxy, hétéroaryle et hétéroaryloxy ;
R⁵ : l'hydrogène,
un groupe alkyle en C1-C10, cycloalkyle en C3-C6, alcényle en C2-C10, alcynyle en C2-C10, (alkyle en C1-C10)carbonyle, (alcényle en C2-C10)carbonyle, (alcynyle en C3-C10)carbonyle ou alkylsulfonyle en C1-C10; ces groupes pouvant être halogénés en totalité ou en partie ou pouvant porter un à trois des substituants suivants : cyano, nitro, hydroxy, mercapto, amino, carboxyle, aminocarbonyle, aminothiocarbonyle, halogéno, alkyle en C1-C6, halogénoalkyle en C1-C6, alkylsulfonyle en C1-C6, alkylsulfoxyle en C1-C6, alcoxy en C1-C6, halogénoalcoxy en C1-C6, (alcoxy en C1-C6)carbonyle, alkylthio en C1-C6, alkylamino en C1-C6, di-(alkyle en C1-C6)amino, (alkyle en C1-C6)aminocarbonyle, di-(alkyle en C1-C6)aminocarbonyle, (alkyle en C1-C6)aminothiocarbonyle, di-(alkyle en C1-C6)aminothiocarbonyle, alcényle en C2-C6, alcényloxy en C2-C6, cycloalkyle en C3-C6, cycloalkyloxy en C3-C6, hétérocyclyle, hétérocyclyloxy, benzyle, benzyloxy, aryle, aryloxy, arylthio, hétéroaryle, hétéroaryloxy et hétéroarylthio, les groupes cycliques pouvant être eux-mêmes halogénés en totalité ou en partie ou pouvant porter un à trois des substituants suivants : cyano, nitro, hydroxy, mercapto, amino, carboxyle, aminocarbonyle, aminothiocarbonyle, halogéno, alkyle en C1-C6, halogénoalkyle en C1-C6, alkylsulfonyle en C1-C6, alkylsulfoxyde en C1-C6, cycloalkyle en C3-C6, alcoxy en C1-C6, halogénoalcoxy en C1-C6, (alkyle en Cl-C6)oxycarbonyle, alkylthio en C1-C6, alkylamino en C1-C6, di-(alkyle en C1-C6)amine, (alkyle en C1-C6)aminocarbonyle, di-(alkyle en C1-C6)aminocarbonyle, (alkyle en C1-C6)aminothiocarbonyle, di-(alkyle en Cl-C6)aminothiocarbonyle, alcényle en C2-C6, alcényloxy en C2-C6, benzyle, benzyloxy, aryle, aryloxy, arylthio, hétéroayle, hétéroaryloxy, hétéroarylthio ou C(=NOR⁶)-Aₘ-R⁷ ;
un groupe aryle, arylcarbonyle, arylsulfonyle, hétéroaryle, hétéroarylcarbonyle ou hétéroarylsulfonyle, ces groupes pouvant être halogénés en totalité ou en partie ou pouvant porter un à trois des substituants suivants : cyano, nitro, hydroxy, mercapto, amino, carboxyle, aminocarbonyle, aminothiocarbonyle, halogéno, alkyle en C1-C6, halogénoalkyle en C1-C6, (alkyle en C1-C6)carbonyle, alkylsulfonyle en C1-C6, alkylsulfoxyde en C1-C6, cycloalkyle en C3-C6, alcoxy en C1-C6, halogénoalcoxy en C1-C6, (alkyle en C1-C6)oxycarbonyle, alkylthio en C1-C6, alkylamino en C1-C6, di-(alkyle en C1-C6)amine, (alkyle en C1-C6)aminocarbonyle, di-(alkyle en C1-C6)aminocarbonyle, (alkyle en C1-C6)aminothiocarbonyle, di-(alkyle en C1-C6)aminothiocarbonyle, alcényle en C2-C6, alcényloxy en C2-C6, benzyle, benzyloxy, aryle, aryloxy, hétéroaryle, hétéroaryloxy ou C(=NOR⁶)-Aₘ-R⁷) ;
et
A représente l'oxygène, le soufre ou l'azote, ce dernier pouvant porter de l'hydrogène ou un groupe alkyle en C1-C6 ;
m est égal à O ou 1 ;
R⁶ représente l'hydrogène, un groupe alkyle en C1-C6, alcényle en C2-C6 ou alcynyle en C2-C6 et
R⁷ représente l'hydrogène, un groupe alkyle en C1-C6, alcényle en C2-C6 ou alcynyle en C2-C6,
et leurs sels.

2. Composés de formule I de la revendication 1, pour lesquels n est égal à 0.

3. Composés de formule I de la revendication 1, pour lequels R¹ représente un groupe méthyle.

4. Procédé de préparation des composés de formule I de la revendication 1, dans laquelle R a une signification autre que l'hydrogène et R³ une signification autre qu'un halogène, caractérisé par le fait que l'on fait réagir un dérivé benzylique de formule II 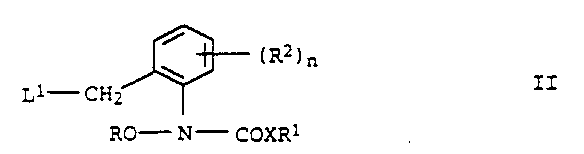 dans laquelle L¹ représente un groupe éliminable par échange nucléophile, de manière connue en soi, avec une oxime de formule III
R⁵ON= C(R⁴) - C(R³) = NOH III

5. Procédé de préparation des composés de formule I de la revendication 1 dans laquelle R a une signification autre que l'hydrogène et R³ et R⁴ des significations autres que des halogènes, caractérisé par le fait que l'on fait réagir un dérivé benzylique de formule II de la revendication 1, de manière connue en soi, avec une dioxime de formule IV
HON= C(R⁴) - C(R³) = NOH IV
ce qui donne un composé de formule V 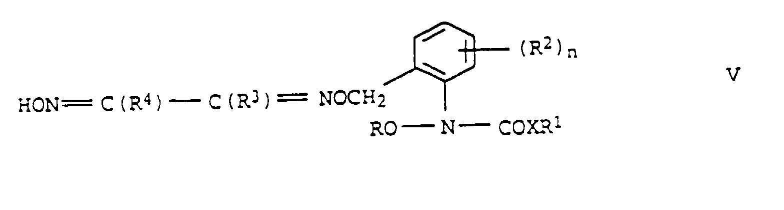 qu'on fait ensuite réagir avec un composé de formule VI
R⁵ - L² VI
dans laquelle L² représente un groupe éliminable par échange nucléophile, ce qui donne un composé I.

6. Procédé de préparation des composés de formule I de la revendication 1 dans laquelle R³ a une signification autre qu'un halogène, caractérisé par le fait que l'on fait réagir un dérivé benzylique de formule II de la revendication 1, de manière connue en soi, avec une céto-oxime de formule VII
O = C(R⁴) - C(R³) = NOH VII
ce qui donne un composé de formule VIII 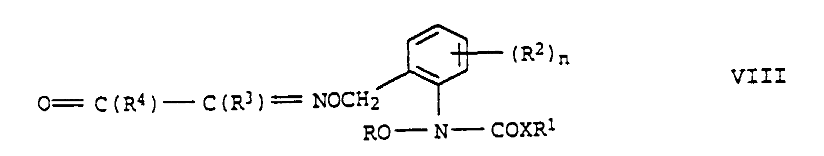 qu'on fait réagir ensuite, soit
a) d'abord avec l'hydroxylamine ou l'un de ses sels puis avec un composé de formule VI (R⁵-L²) selon la revendication 5,
soit
b) avec une hydroxylamine ou un sel d'hydroxylammonium de formule IXa ou IXb respectivement
R⁵ - ONH₂ IXa
R⁵ - ONH₃^{⊕} Q^{⊖} IXb
dans lesquelles Q^{θ} représente l'anion d'un acide, ce qui donne I dans les deux cas.

7. Produit destiné à combattre les parasites animaux ou les mycètes nuisibles, contenant des additifs usuels et une quantité efficace d'un composé de formule I de la revendication 1.

8. Produit selon la revendication 7, pour combattre les parasites animaux de la classe des insectes, des arachnides ou des nématodes.

9. Procédé pour combattre les parasites animaux ou les mycètes nuisibles, caractérisé par le fait que l'on traite les parasites ou les mycètes nuisibles, leur habitat ou bien les végétaux, surfaces, matériaux ou locaux qu'on veut en protéger par une quantité efficace d'un composé de formule I de la revendication 1.

10. Utilisation des composés I de la revendication 1 pour la préparation de produits prévus pour combattre les parasites animaux ou les mycètes nuisibles.

11. Utilisation des composés I de la revendication pour la lutte contre les parasites animaux ou les mycètes nuisibles.

12. Composés de formule VIII de la revendication 6.

13. Utilisation des composés de formule VIII de la revendication 6 en tant que produits intermédiaires.

14. Composés de formule X 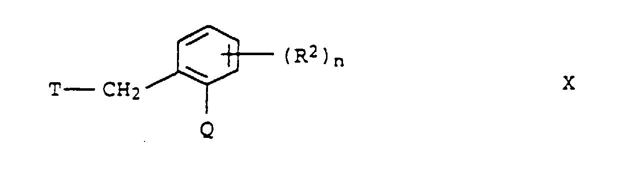 dans laquelle l'indice et les substituants ont les significations suivantes :
Q : NO₂, NHOH et N(OR)-CO₂C₆H₅ ;
T : O=C(R⁴)-C(R³)=NO-, HON=C(R⁴)-C(R³)=NO- ou R⁵ON=C(R⁴)-C(R³)=NO-,
les symboles R, R¹, R², R³, R⁴ et R⁵ et l'indice n ayant les significations indiquées dans la revendication 1.

15. Utilisation des composés de formule X de la revendication 14 en tant que produits intermédiaires.

16. Composés de formule XI 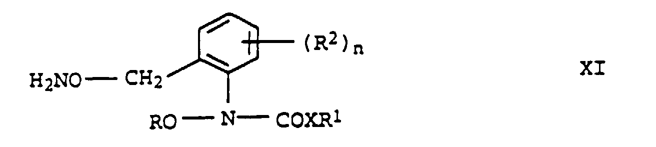 dans laquelle l'indice n et les symboles R, R¹ et R² ont les significations indiquées dans la revendication 1.

17. Utilisation des composés de formule XI de la revendication 16 en tant que produits intermédiaires.
